# EUROPÄISCHE PATENTSCHRIFT

(11) **EP 0 835 256 B1**
(45) Veröffentlichungstag und Bekanntmachung des Hinweises auf die Patenterteilung: **02.01.2002**
(21) Anmeldenummer: 96922821.2
(22) Anmeldetag: 17.06.1996
(51) Int. Cl.: C07D 491/04, C07D 413/12, C07D 409/12, C07D 417/12, C07D 413/06, C07D 403/12, C07D 403/06, C07D 401/12, A01N 43/90, A01N 43/647, A01N 43/72

(54) **MIKROBIZIDE BENZOTRIAZOLE**
MICROBICIDAL BENZOTRIAZOLES
BENZOTRIAZOLES MICROBICIDES

(30) Priorität: 28.06.1995 DE 19523446
(43) Veröffentlichungstag der Anmeldung: 15.04.1998
(73) Patentinhaber: BAYER AG, 51368 Leverkusen (DE)
(72) Erfinder: ASSMANN, Lutz, D-23701 Eutin (DE); TIEMANN, Ralf, D-51375 Leverkusen (DE); KUGLER, Martin, D-42799 Leichlingen (DE); SCHRAGE, Heinrich, D-47829 Krefeld (DE); STENZEL, Klaus, D-40595 Düsseldorf (DE)
(86) Internationale Anmeldenummer: EP9602611
(87) Internationale Veröffentlichungsnummer: WO9701561

(56) Entgegenhaltungen:
- EP-A- 0 238 824
- EP-A- 0 430 471
- EP-A- 0 462 931
- EP-A- 0 608 753
- WO-A-94/25446
- DE-B- 1 046 937
- US-A- 3 740 396
- ARZNEIMITTEL-FORSCHUNG, Bd. 31, Nr. 5, 1981, AULENDORF DE, Seiten 747-52, XP002013077 N. NEYKOVA ET AL.: "Benzoxazolone-5-sulphonanilides, 1-(benzoxazolone-5'-sulphonyl)-benzo- triazoles and 4-hydroxy-3,2'-diamino- benzenesulphonanilides with antiviral activity"
- CHEMISCHE BERICHTE, Bd. 103, Nr. 9, 1.September 1970, WEINHEIM DE, Seiten 2828-35, XP002013078 A.J. HUBERT ET AL.: "Thermolyse und Photolyse von Benzotriazolyl-(1)-Derivaten"
- JOURNAL OF THE CHEMICAL SOCIETY (C), 1969, LONDON GB, Seiten 1334-6, XP002013079 A.J. HUBERT: "Photochemistry of benzotriazoles"
- JOURNAL OF HETEROCYCLIC CHEMISTRY, Bd. 24, Nr. 5, 1987, PROVO US, Seiten 1301-3, XP002013080 C. CORRAL ET AL.: "Reactions of methyl 3-hydroxythiophene-2-carboxylate. Part 4. Synthesis of methyl 5-azolyl-3- hydroxythiophene-2-carboxylates"
- HETEROCYCLES, Bd. 36, Nr. 6, 1.Juni 1993, TOKYO JP, Seiten 1253-62, XP002013081 A.R. KATRITZKY ET AL.: "N-Sulfonylbenzotriazoles. Part 2. Reactions of 1,1'-sulfonyldibenzotriazole and 1-benzenesulfonyl-1,2,4-triazole with alcohols; a new approach to N-alkylbenzotriazoles and N-alkyl-1,2,4-triazoles"
- JOURNAL OF HETEROCYCLIC CHEMISTRY, Bd. 31, Nr. 4, 1994, PROVO US, Seiten 757-63, XP002013082 A.R. KATRITZKY ET AL.: "Chemistry of alpha-(benzotriazol-1-yl)alkylpyridines. Unusual nucleophilic attack at C-3a at the benzotriazole ring"
- TETRAHEDRON, Bd. 48, Nr. 37, 11.September 1992, OXFORD GB, Seiten 7817-22, XP002013083 A.R. KATRITZKY ET AL.: "Sulfonyl derivatives of benzotriazole: Part 1. A novel approach to the activation of carboxylic acids"
- DATABASE CROSSFIRE Beilstein Informationssysteme GmbH, Frankfurt DE XP002013084 & ARCH. PHARM., Bd. 321, 1988, Seiten 463-7,
- DATABASE CROSSFIRE Beilstein Informationssysteme GmbH, Frankfurt DE XP002013085 & J. CHEM. SOC., Bd. 127, 1925, Seite 270, 2707
- DATABASE CROSSFIRE Beilstein Informationssysteme GmbH, Frankfurt DE XP002013086 & ARCH. PHARM. BER. DTSCH. PHARM. GES., Bd. 303, Nr. 4, 1970, Seiten 310-7,

## Beschreibung

Die vorliegende Erfindung betrifft neue Benzotriazole, ein Verfahren zu ihrer Herstellung und deren Verwendung als Mikrobizide im Pflanzenschutz und im Materialschutz.

Es ist bereits bekannt geworden, daß bestimmte Benzotriazol-Derivate fungizide Eigenschaften besitzen (vgl. DE-A 1 046 937, EP-A 0 238 824, EP-A 0 462 931, EP-A 0 549 532 und US-A 2 943 017). Die Wirksamkeit der dort beschriebenen Stoffe ist gut, läßt aber bei niedrigen Aufwandmengen in manchen Fällen zu wünschen übrig.

Es wurden nun neue Benzotriazole der Formel 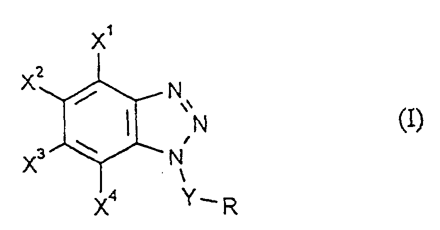 in welcher
X¹, X², X³ und X⁴ unabhängig voneinander für Wasserstoff, Fluor, Chlor, Brom, Iod, Cyano, Nitro, geradkettiges oder verzweigtes Alkyl mit 1 bis 8 Kohlenstoffatomen, geradkettiges oder verzweigtes Halogenalkyl mit 1 bis 6 Kohlenstoffatomen und 1 bis 13 gleichen oder verschiedenen Halogenatomen, geradkettiges oder verzweigtes Alkoxy mit 1 bis 8 Kohlenstoffatomen, geradkettiges oder verzweigtes Halogenalkoxy mit 1 bis 6 Kohlenstoffatomen und 1 bis 13 gleichen oder verschiedenen Halogenatomen, geradkettiges oder verzweigtes Alkylthio mit 1 bis 8 Kohlenstoffatomen, geradkettiges oder verzweigtes Halogenalkylthio mit 1 bis 6 Kohlenstoffatomen und 1 bis 13 gleichen oder verschiedenen Halogenatomen, geradkettiges oder verzweigtes Alkylsulfinyl mit 1 bis 8 Kohlenstoffatomen, geradkettiges oder verzweigtes Halogenalkylsulfinyl mit 1 bis 6 Kohlenstoffatomen und 1 bis 13 gleichen oder verschiedenen Halogenatomen, geradkettiges oder verzweigtes Alkylsulfonyl mit 1 bis 8 Kohlenstoffatomen, geradkettiges oder verzweigtes Halogenalkylsulfonyl mit 1 bis 6 Kohlenstoffatomen und 1 bis 13 gleichen oder verschiedenen Halogenatomen, gegebenenfalls einfach bis fünffach, gleichartig oder verschieden durch Halogen und/oder Alkyl mit 1 bis 4 Kohlenstoffatomen substituiertes Cycloalkyl mit 3 bis 6 Kohlenstoffatomen, für Hydroxycarbonyl, Alkylcarbonyl mit 1 bis 6 Kohlenstoffatomen im geradkettigen oder verzweigten Alkylteil, Alkoxycarbonyl mit 1 bis 6 Kohlenstoffatomen im geradkettigen oder verzweigten Alkoxyteil, Cycloalkylcarbonyl mit 3 bis 6 Kohlenstoffatomen im Cycloalkylteil, Cycloalkoxycarbonyl mit 3 bis 6 Kohlenstoffatomen im Cycloalkylteil, für 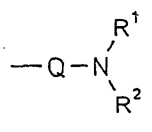 oder -Z-R³ stehen,
worin
R¹ und R² unabhängig voneinander für Wasserstoff, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, geradkettiges oder verzweigtes Alkoxyalkyl mit 1 bis 4 Kohlenstoffatomen im Alkoxyteil und 1 bis 4 Kohlenstoffatomen im Alkylteil, Alkylcarbonyl mit 1 bis 6 Kohlenstoffatomen im geradkettigen oder verzweigten Alkylteil, Aryl mit 6 bis 10 Kohlenstoffatomen, Arylcarbonyl mit 6 bis 10 Kohlenstoffatomen im Arylteil stehen, Arylsulfonyl mit 6 bis 10 Kohlenstoffatomen, Arylaminocarbonyl mit 6 bis 10 Kohlenstoffatomen im Arylteil oder für Aryl methylsulfonyl mit 6 bis 10 Kohlenstoffatomen im Arylteil stehen, wobei jeder der zuvor genannten Aryl-Reste einfach bis dreifach, gleichartig oder verschieden substituiert sein kann durch Halogen, Cyano, Nitro, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Halogenalkylthio mit 1 bis 4 Kohlenstoffatomen und 1 bis 5 gleichen oder verschiedenen Halogenatomen, Alkylsulfinyl mit 1 bis 4 Kohlenstoffatomen, Alkylsulfonyl mit 1 bis 4 Kohlenstoffatomen, Halogenalkylsulfinyl mit 1 bis 4 Kohlenstoffatomen und 1 bis 5 gleichen oder verschiedenen Halogenatomen und/oder Halogenalkylsulfonyl mit 1 bis 4 Kohlenstoffatomen und 1 bis 5 gleichen oder verschiedenen Halogenatomen,
oder
R¹ und R² auch gemeinsam mit dem Stickstoffatom, an das sie gebunden sind, vorzugsweise für einen gegebenenfalls einfach bis dreifach durch Alkyl mit 1 bis 4 Kohlenstoffatomen substituierten heterocyclischen Ring mit 5 oder 6 Ringgliedern stehen, der noch ein Sauerstoffatom oder eine C₁-C₄-Alkyliminogruppe enthalten kann,
Q für eine direkte Bindung oder für eine Carbonyl-Gruppe steht und
R³ für Aryl mit 6 bis 10 Kohlenstoffatomen steht, wobei jeder dieser Reste einfach bis dreifach, gleichartig oder verschieden substituiert sein kann durch Halogen, Cyano, Nitro, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Halogenalkylthio mit 1 bis 4 Kohlenstoffatomen und 1 bis 5 gleichen oder verschiedenen Halogenatomen, Alkylsulfinyl mit 1 bis 4 Kohlenstoffatomen, Alkylsulfonyl mit 1 bis 4 Kohlenstoffatomen, Halogenalkylsulfinyl mit 1 bis 4 Kohlenstoffatomen und 1 bis 5 gleichen oder verschiedenen Halogenatomen und/oder Halogenalkylsulfonyl mit 1 bis 4 Kohlenstoffatomen und 1 bis 5 gleichen oder verschiedenen Halogenatomen, oder
R³ für einen gesättigten oder ungesättigten Heterocyclyl-Rest mit 5 oder 6 Ringgliedern und 1 bis 3 Heteroatomen steht, wobei diese Reste einfach bis dreifach, gleichartig oder verschieden substituiert sein können durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Alkoxycarbonyl mit 1 bis 3 Kohlenstoffatomen im Alkoxyteil, Cycloalkyl mit 3 bis 6 Kohlenstoffatomen, Cyano und/oder Nitro,
Z für eine direkte Bindung sowie für CH₂, O, S, SO, SO₂, CO, oder
   für -CO-O- steht, wobei das Sauerstoffatom mit dem Aryl- bzw. Heterocyclyl-Rest verbunden ist, oder
   für -SO₂-O- steht, wobei das Schwefelatom mit dem Aryl- bzw. Heterocyclyl-Rest verbunden ist, oder
   für -S-CH₂-SO₂- steht, wobei das Schwefelatom der Thio-Gruppe mit dem Aryl- bzw. Heterocyclyl-Rest verbunden ist,
oder
X²und X³ gemeinsam für eine gegebenenfalls einfach bis sechsfach durch Halogen, Alkyl mit 1 bis 4 Kohlenstoffatomen und/oder Halogenalkyl mit 1 bis 4 Kohlenstoffatomen und 1 bis 9 Halogenatomen substituierte Alkylenkette mit 3 oder 4 Gliedern stehen, in der ein oder zwei (nicht benachbarte) Kohlenstoffatome durch Sauerstoffatome ersetzt sein können, oder
X² und X³ gemeinsam für einen Rest der Formel 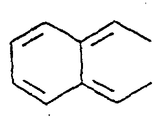 stehen,
R für einen gesättigten oder ungesättigten Heterocyclyl-Rest mit 5 oder 6 Ringgliedern und 1 bis 3 Heteroatomen steht, wobei diese Reste einfach bis dreifach, gleichartig oder verschieden substituiert sein können durch Halogen, Cyano, Nitro, Hydroxy, Amino, Formyl, Carboxy, Carbamoyl, Thiocarbamoyl, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Alkoxycarbonyl mit 1 bis 4 Kohlenstoffatomen im Alkoxyteil, Cycloalkyl mit 3 bis 6 Kohlenstoffatomen, Halogenalkylsulfinyl mit 1 bis 4 Kohlenstoffatomen und 1 bis 5 gleichen oder verschiedenen Halogenatomen, Halogenalkylsulfonyl mit 1 bis 4 Kohlenstoffatomen und 1 bis 5 gleichen oder verschiedenen Halogenatomen, Alkylamino mit 1 bis 4 Kohlenstoffatomen, Hydroxyalkylamino mit 1 bis 4 Kohlenstoffatomen, Dialkylamino mit 1 bis 4 Kohlenstoffatomen in jeder Alkylgruppe, Alkylcarbonyl mit 1 bis 4 Kohlenstoffatomen im Alkylteil, Alkylcarbonylamino mit 1 bis 4 Kohlenstoffatomen im Alkylteil, Hydroxyiminoalkyl mit 1 bis 4 Kohlenstoffatomen im Alkylteil, Alkoximinoalkyl mit 1 bis 4 Kohlenstoffatomen im Alkoxyteil und 1 bis 4 Kohlenstoffatomen im Alkylteil, Alkylcarbonyloxy mit 1 bis 4 Kohlenstoffatomen im Alkylteil und/oder Halogenalkylcarbonyloxy mit 1 bis 4 Kohlenstoffatomen in der Halogenalkylgruppe und 1 bis 5 gleichen oder verschiedenen Halogenatomen, und wobei die Heterocyclyl-Reste auch Oxo-Gruppen enthalten können,
und
Y für -SO₂- steht,
sowie deren Säureadditions-Salze und Metallsalz-Komplexe gefunden.

Weiterhin wurde gefunden, daß man Benzotriazole der Formel (I) sowie deren Säureadditions-Salze und Metallsalz-Komplexe erhält, wenn man Benzotriazole der Formel 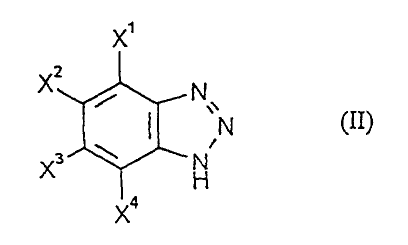 in welcher
X¹, X², X³ und X⁴ die oben angegebenen Bedeutungen haben,
mit Halogeniden der Formel

   Hal-Y-R (III)

   in welcher
R und Y die oben angegebenen Bedeutungen haben und
Hal für Chlor oder Brom steht,
gegebenenfalls in Gegenwart eines Säurebindemittels und gegebenenfalls in Gegenwart eines Verdünnungsmittels umsetzt und gegebenenfalls an die so erhaltenen Verbindungen der Formel (I) eine Säure oder ein Metallsalz addiert.

Schließlich wurde gefunden, daß die Benzotriazole der Formel (I) sowie deren Säureadditions-Salze und Metallsalz-Komplexe sehr gute mikrobizide Eigenschaften besitzen und sowohl im Pflanzenschutz als auch im Materialschutz eingesetzt werden können.

Überraschenderweise zeigen die erfindungsgemäßen Stoffe eine deutlich bessere fungizide Wirksamkeit als die konstitutionell ähnlichsten vorbekannten Substanzen gleicher Indikation.

Die erfindungsgemäßen Stoffe sind durch die Formel (I) allgemein definiert.
X¹, X², X³ und X⁴ stehen unabhängig voneinander bevorzugt für Wasserstoff, Fluor, Chlor, Brom, Iod, Cyano, Nitro, geradkettiges oder verzweigtes Alkyl mit 1 bis 6 Kohlenstoffatomen, Halogenalkyl mit 1 oder 2 Kohlenstoffatomen und 1 bis 5 Fluor-, Chlor- und/oder Bromatomen, geradkettiges oder verzweigtes Alkoxy mit 1 bis 6 Kohlenstoffatomen, Halogenalkoxy mit 1 oder 2 Kohlenstoffatomen und 1 bis 5 Fluor-, Chlor- und/oder Bromatomen, geradkettiges oder verzweigtes Alkylthio mit 1 bis 6 Kohlenstoffatomen, Halogenalkylthio mit 1 oder 2 Kohlenstoffatomen und 1 bis 5 Fluor-, Chlor- und/oder Bromatomen, geradkettiges oder verzweigtes Alkylsulfinyl mit 1 bis 6 Kohlenstoffatomen, Halogenalkylsulfinyl mit 1 oder 2 Kohlenstoffatomen und 1 bis 5 Fluor-, Chlor- und/oder Bromatomen, geradkettiges oder verzweigtes Alkylsulfonyl mit 1 bis 6 Kohlenstoffatomen, Halogenalkylsulfonyl mit 1 oder 2 Kohlenstoffatomen und 1 bis 5 Fluor-, Chlor- und/oder Bromatomen, gegebenenfalls einfach bis dreifach, gleichartig oder verschieden durch Fluor, Chlor, Brom, Methyl und/oder Ethyl substituiertes Cycloalkyl mit 3 bis 6 Kohlenstoffatomen, für Hydroxycarbonyl, Alkylcarbonyl mit 1 bis 4 Kohlenstoffatomen im geradkettigen oder verzweigten Alkylteil, Alkoxycarbonyl mit 1 bis 4 Kohlenstoffatomen im geradkettigen oder verzweigten Alkoxyteil, Cycloalkylcarbonyl mit 3 bis 6 Kohlenstoffatomen im Cycloalkylteil, Cycloalkoxycarbonyl mit 3 bis 6 Kohlenstoffatomen im Cycloalkylteil, für 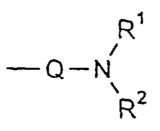 oder -Z-R³.
R¹ und R² stehen unabhängig voneinander bevorzugt für Wasserstoff, geradkettiges oder verzweigtes Alkyl mit 1 bis 4 Kohlenstoffatomen, Halogenalkyl mit 1 oder 2 Kohlenstoffatomen und 1 bis 5 Fluor-, Chlor-und/oder Bromatomen, geradkettiges oder verzweigtes Alkoxyalkyl mit 1 bis 3 Kohlenstoffatomen im Alkoxyteil und 1 bis 3 Kohlenstoffatomen im Alkylteil, Alkylcarbonyl mit 1 bis 4 Kohlenstoffatomen im geradkettigen oder verzweigten Alkylteil, Phenyl, Phenylcarbonyl, Phenylsulfonyl, Phenylaminocarbonyl oder Phenylmethylsulfonyl, wobei jeder der zuvor genannten Phenylreste einfach bis dreifach, gleichartig oder verschieden substituiert sein kann durch Fluor, Chlor, Brom, Cyano, Nitro, Alkyl mit 1 oder 2 Kohlenstoffatomen, Alkoxy mit 1 oder 2 Kohlenstoffatomen, Alkylthio mit 1 oder 2 Kohlenstoffatomen, Halogenalkyl mit 1 oder 2 Kohlenstoffatomen und 1 bis 5 Fluor-, Chlor- und/oder Bromatomen, Halogenalkoxy mit 1 oder 2 Kohlenstoffatomen und 1 bis 5 Fluor-, Chlor- und/oder Bromatomen, Halogenalkylthio mit 1 oder 2 Kohlenstoffatomen und 1 bis 5 Fluor-, Chlor- und/oder Bromatomen, Alkylsulfinyl mit 1 oder 2 Kohlenstoffatomen, Alkylsulfonyl mit 1 oder 2 Kohlenstoffatomen, Halogenalkylsulfinyl mit 1 oder 2 Kohlenstoffatomen und 1 bis 5 Fluor-, Chlor- und/oder Bromatomen und/oder Halogenalkylsulfonyl mit 1 oder 2 Kohlenstoffatomen und 1 bis 5 Fluor-, Chlor- und/oder Bromatomen.
R¹ und R² stehen außerdem auch gemeinsam mit dem Stickstoffatom, an das sie gebunden sind, bevorzugt für einen gegebenenfalls einfach bis dreifach durch Methyl und/oder Ethyl substituierten, gesättigten heterocyclischen Ring mit 5 oder 6 Ringgliedern, wobei ein Kohlenstoffatom des Ringes durch Sauerstoff oder Methylimino ersetzt sein kann.
Q steht auch bevorzugt für eine direkte Bindung oder für eine Carbonylgruppe.
R³ steht bevorzugt für Phenyl, das einfach bis dreifach, gleichartig oder verschieden substituiert sein kann durch Fluor, Chlor, Brom, Cyano, Nitro, Alkyl mit 1 oder 2 Kohlenstoffatomen, Alkoxy mit 1 oder 2 Kohlenstoffatomen, Alkylthio mit 1 oder 2 Kohlenstoffatomen, Halogenalkyl mit 1 oder 2 Kohlenstoffatomen und 1 bis 5 Fluor-, Chlor- und/oder Bromatomen, Halogenalkoxy mit 1 oder 2 Kohlenstoffatomen und 1 bis 5 Fluor-, Chlor- und/oder Bromatomen, Halogenalkylthio mit 1 oder 2 Kohlenstoffatomen und 1 bis 5 Fluor-, Chlor- und/oder Bromatomen, Alkylsulfinyl mit 1 oder 2 Kohlenstoffatomen, Alkylsulfonyl mit 1 oder 2 Kohlenstoffatomen, Halogenalkylsulfinyl mit 1 oder 2 Kohlenstoffatomen und 1 bis 5 Fluor-, Chlor- und/oder Bromatomen und/oder Halogenalkylsulfonyl mit 1 oder 2 Kohlenstoffatomen und 1 bis 5 Fluor-, Chlor- und/oder Bromatomen, oder
R³ steht bevorzugt für einen gesättigten oder ungesättigten Heterocyclyl-Rest mit 5 oder 6 Ringgliedern und 1 bis 3 Heteroatomen, wie Stickstoff, Sauerstoff und/oder Schwefel, wobei diese Reste einfach bis dreifach, gleichartig oder verschieden substituiert sein können durch Fluor, Chlor, Brom, Alkyl mit 1 oder 2 Kohlenstoffatomen, Alkoxy mit 1 oder 2 Kohlenstoffatomen, Halogenalkyl mit 1 oder 2 Kohlenstoffatomen und 1 bis 5 Fluor-, Chlor- und/oder Bromatomen, Halogenalkoxy mit 1 oder 2 Kohlenstoffatomen und 1 bis 5 Fluor-, Chlor- und/oder Bromatomen, Alkoxycarbonyl mit 1 oder 2 Kohlenstoffatomen im Alkoxyteil, Cycloalkyl mit 3 bis 6 Kohlenstoffatomen, Cyano und/oder Nitro.
Z steht auch bevorzugt für eine direkte Bindung sowie für CH₂, O, S, SO, SO₂, CO, oder
   für -CO-O-, wobei das Sauerstoffatom mit dem Phenyl- bzw. Heterocyclyl-Rest verbunden ist, oder
   für -SO₂-O-, wobei das Schwefelatom mit dem Phenyl- bzw. Heterocyclyl-Rest verbunden ist, oder
   für -S-CH₂-SO₂-, wobei das Schwefelatom der Thiogruppe mit dem Phenyl- bzw. Heterocyclyl-Rest verbunden ist.
X² und X³ stehen auch gemeinsam bevorzugt für eine gegebenenfalls einfach bis sechsfach durch Fluor, Chlor, Methyl und/oder Trifluormethyl substituierte Alkylenkette mit 3 oder 4 Gliedern, in der ein oder zwei (nicht benachbarte) Kohlenstoffatome durch Sauerstoff ersetzt sein können, oder
X² und X³ stehen gemeinsam für einen Rest der Formel 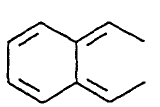
R steht bevorzugt für einen gesättigten oder ungesättigten Heterocyclyl-Rest mit 5 oder 6 Ringgliedern und 1 bis 3 Heteroatomen, wie Stickstoff, Sauerstoff und/oder Schwefel, wobei diese Reste einfach bis dreifach, gleichartig oder verschieden substituiert sein können durch Fluor, Chlor, Brom, Cyano, Nitro, Hydroxy, Amino, Formyl, Carboxy, Carbamoyl, Thiocarbamoyl, Alkyl mit 1 bis 4 Kohlenstoffatomen, Alkoxy mit 1 bis 4 Kohlenstoffatomen, Halogenalkyl mit 1 oder 2 Kohlenstoffatomen und 1 bis 5 Fluor-, Chlor-und/oder Bromatomen, Halogenalkoxy mit 1 oder 2 Kohlenstoffatomen und 1 bis 5 Fluor-, Chlor- und/oder Bromatomen, Alkoxycarbonyl mit 1 oder 2 Kohlenstoffatomen im Alkoxyteil, Cycloalkyl mit 3 bis 6 Kohlenstoffatomen, Halogenalkylsulfinyl mit 1 oder 2 Kohlenstoffatomen und 1 bis 5 Fluor-, Chlor- und/oder Bromatomen, Halogenalkylsulfonyl mit 1 oder 2 Kohlenstoffatomen und 1 bis 5 Fluor-, Chlor- und/oder Bromatomen, Alkylamino mit 1 oder 2 Kohlenstoffatomen, Hydroxyalkylamino mit 1 oder 2 Kohlenstoffatomen, Dialkylamino mit 1 oder 2 Kohlenstoffatomen in jeder Alkylgruppe, Alkylcarbonyl mit 1 oder 2 Kohlenstoffatomen im Alkylteil, Alkylcarbonylamino mit 1 bis 3 Kohlenstoffatomen im Alkylteil, Hydroximinoalkyl mit 1 oder 2 Kohlenstoffatomen im Alkylteil, Alkoximinoalkyl mit 1 oder 2 Kohlenstoffatomen im Alkoxyteil und 1 oder 2 Kohlenstoffatomen im Alkylteil, Alkylcarbonyloxy mit 1 oder 2 Kohlen-stoffatomen in der Alkylgruppe und/oder Halogenalkylcarbonyloxy mit 1 oder 2 Kohlenstoffatomen in der Halogenalkylgruppe und 1 bis 5 Fluor-, Chlor- und/oder Bromatomen,
   und wobei die Heterocyclyl-Reste auch eine oder zwei Oxo-Gruppen enthalten können.
Y steht für SO₂.
X¹, X², X³ und X⁴ stehen unabhängig voneinander besonders bevorzugt für Wasserstoff, Fluor, Chlor, Brom, Cyano, Nitro, für Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Trifluormethyl, Difluormethoxy, Trifluormethoxy, Difluormethylthio, Trifluormethylthio, Trifluormethylsulfinyl, Trifluormethylsulfonyl, Acetyl, Acetyloxy, Methoxycarbonyl, Ethoxycarbonyl, Cyclopropyl, Cyclohexyl, für 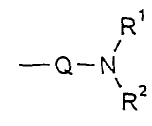 oder -Z-R³.
R¹ und R² stehen unabhängig voneinander besonders bevorzugt für Wasserstoff, Methyl, Ethyl, n-Propyl, Isopropyl oder Phenyl.
R¹ und R² stehen außerdem auch gemeinsam mit dem Stickstoffatom, an das sie gebunden sind, besonders bevorzugt für Pyrrolidinyl, Piperidinyl, Morpholinyl oder 4-Methyl-piperazinyl.
Q steht auch besonders bevorzugt für eine direkte Bindung oder für eine Carbonylgruppe.
R³ steht besonders bevorzugt für Phenyl, das einfach bis dreifach, gleichartig oder verschieden substituiert sein kann durch Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Trifluormethyl, Difluormethoxy, Trifluormethoxy, Difluormethylthio, Trifluormethylthio, Difluormethylsulfinyl und/oder Trifluormethylsulfonyl, oder
R³ steht besonders bevorzugt für Pyrrolyl, Furyl, Thienyl, Pyrazolyl, Imidazolyl, Thiazolyl, Isothiazolyl, Oxazolyl, Isoxazolyl, Oxadiazolyl, Thiadiazolyl, 1,2,3-Triazinyl, 1,2,4-Triazinyl, 1,3,5-Triazinyl, Pyridinyl, Pyrimidinyl, Pyrazinyl oder Pyridazinyl, wobei diese Reste einfach bis dreifach, gleichartig oder verschieden substituiert sein können durch Fluor, Chlor, Methyl, Methoxy, Trifluormethyl und/oder Trifluormethoxy.
Z steht ganz besonders bevorzugt für eine direkte Bindung sowie für CH₂, O, S, SO, SO₂, CO, oder
   für -CO-O-, wobei das Sauerstoffatom mit dem Phenyl- bzw. Heterocyclyl-Rest verbunden ist, oder
   für -SO₂-O-, wobei das Schwefelatom mit dem Phenyl- bzw. Heterocyclyl-Rest verbunden ist, oder
   für -S-CH₂-SO₂-, wobei das Schwefelatom der Thiogruppe mit dem Phenyl- bzw. Heterocyclyl-Rest verbunden ist.
X² und X³ stehen auch gemeinsam besonders bevorzugt für die Gruppierungen -O-CF₂-O-, -O-CF₂-CHF-O-, -O-CHF-CHF-O-, -O-CF₂-CF₂-O-, -O-CF₂-CFCl-O-, -O-CFCl-CFCl-O-, -(CH₂)₃-, -C(CH₃)₂-CH₂-C(CH₃)₂-, oder 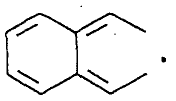
R steht besonders bevorzugt für Pyrrolyl, Furyl, Thienyl, Pyrazolyl, Imidazolyl, Thiazolyl, Isothiazolyl, Oxazolyl, Isoxazolyl, Oxadiazolyl, Thiadiazolyl, 1,2,3-Triazinyl, 1,2,4-Triazinyl, 1,3,5-Triazinyl, Pyridinyl, Pyrimidinyl, Pyrazinyl, Pyridazinyl,
   wobei diese Reste einfach bis dreifach, gleichartig oder verschïeden substituiert sein können durch Fluor, Chlor, Brom, Cyano, Nitro, Amino, Hydroxy, Formyl, Carboxy, Carbamoyl, Thiocarbamoyl, Methyl, Ethyl, n-oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Trifluormethyl, Difluormethoxy, Trifluormethoxy, Difluormethylthio, Trifluormethylthio, Trifluormethylsulfinyl, Trifluormethylsulfonyl, Cyclopropyl, Methylamino, Ethylamino, n- oder i-Propylamino, Dimethylamino, Diethylamino, Acetyl, Propionyl, Methylcarbonylamino, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl und/oder Ethoximinoethyl.
Y steht für SO₂.

Bevorzugte erfindungsgemäße Verbindungen sind auch Additionsprodukte aus Säuren und denjenigen Benzotriazolen der Formel (I), in denen R, X¹, X², X³, X⁴ und Y diejenigen Bedeutungen haben, die für diese Reste als bevorzugt genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure, 1,5-Naphthalindisulfonsäure und außerdem Saccharin und Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Benzotriazolen der Formel (I), in denen R, X¹, X², X³, X⁴ und Y diejenigen Bedeutungen haben, die für diese Reste als bevorzugt genannt wurden.

Hierbei sind Salze des Kupfers, Zinks, Mangans, Magnesiums, Zinns, Eisens-und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Stoffe seien die in den Tabellen 1 bis 8 aufgeführten Benzotriazole genannt.

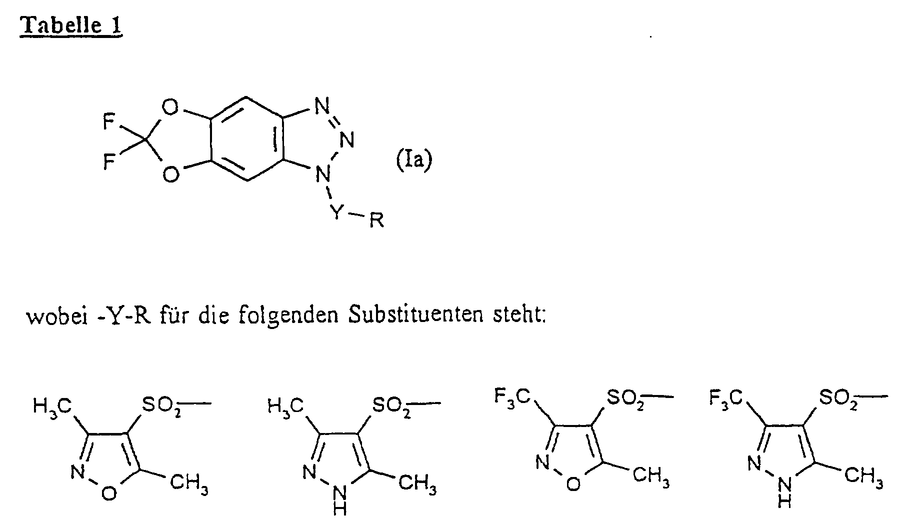

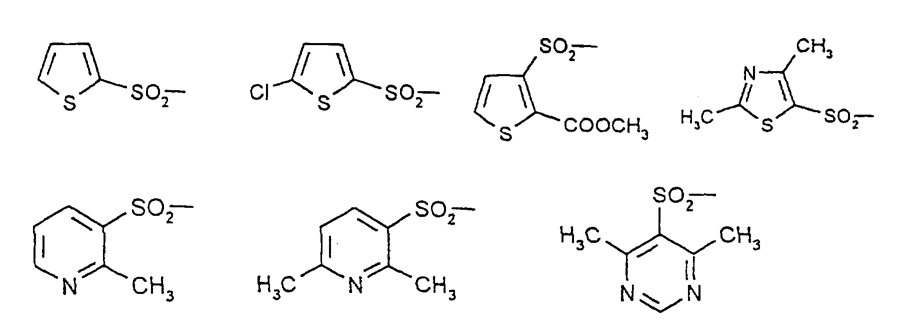

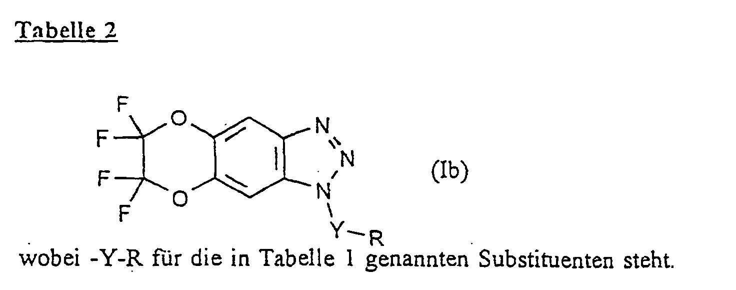

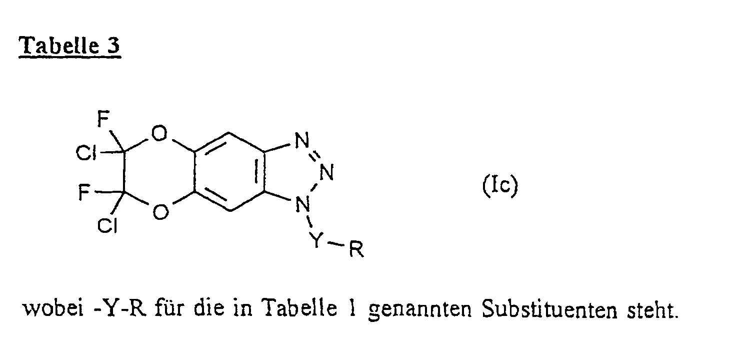

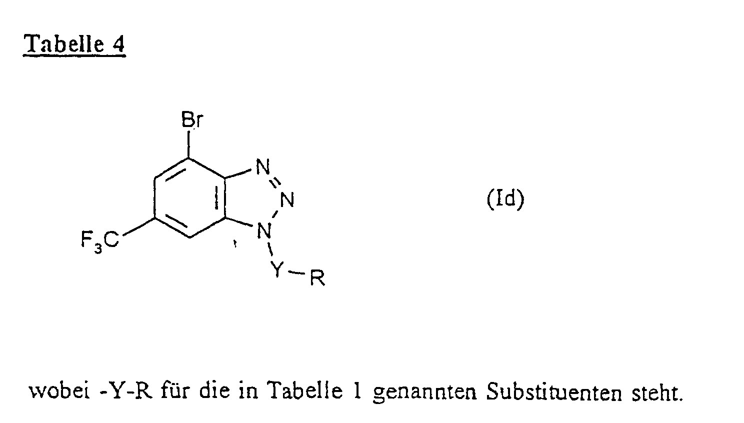

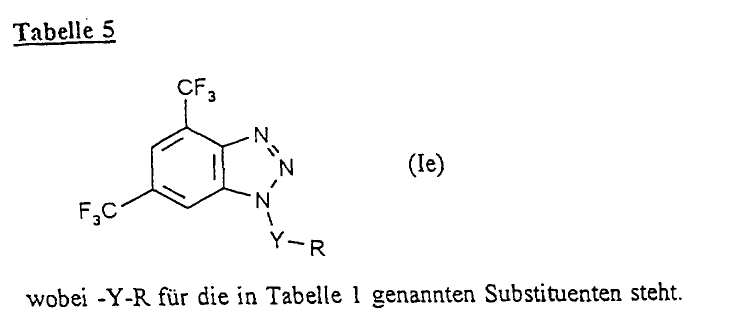

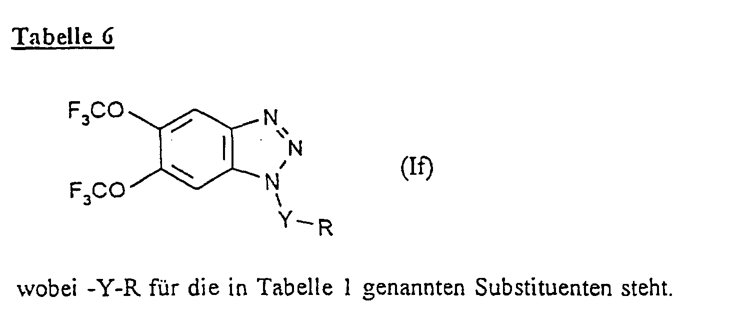

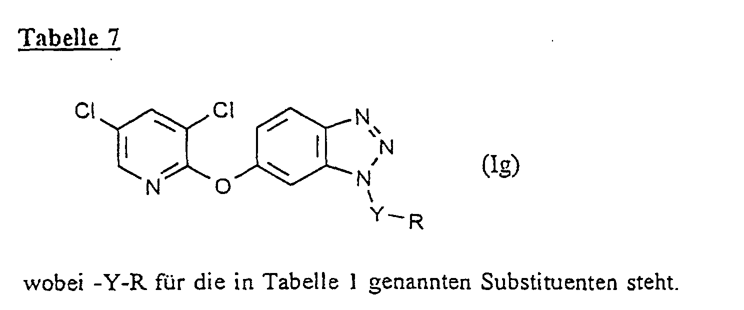

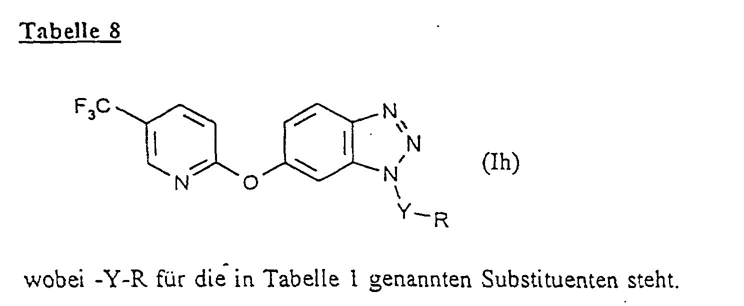

Verwendet man 6,6-Difluor-[1,3]dioxolo-[4,5-f]-1H-benzotriazol und 3,5-Dimethylisoxazol-4-sulfonylchlorid als Ausgangsstoffe, so läßt sich der Verlauf des erfindungsgemäßen Verfahrens durch das folgende Formelschema veranschaulichen:

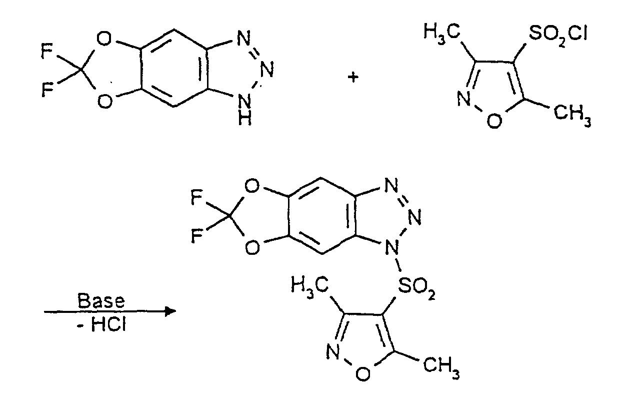

Die zur Durchführung des erfindungsgemäßen Verfahrens als Ausgangsstoffe benötigten Benzotriazole sind durch die Formel (II) allgemein definiert. In dieser Formel haben X¹, X², X³ und X⁴ vorzugsweise diejenigen Bedeutungen, die bereits im Zusammenhang mit der Beschreibung der erfindungsgemäßen Stoffe der Formel (I) für diese Reste als bevorzugt genannt wurden.

Die Benzotriazole der Formel (II) sind bekannt oder lassen sich nach prinzipiell bekannten Methoden herstellen (vgl. Chem. Reviews, 1950, 1; DE-A 3 406 011 und EP-A 0 608 573).

Die außerdem zur Durchführung des erfindungsgemäßen Verfahrens als Ausgangsstoffe benötigten Halogenide sind durch die Formel (III) allgemein definiert. In dieser Formel haben R und Y vorzugsweise diejenigen Bedeutungen, die bereits im Zusammenhang mit der Beschreibung der erfindungsgemäßen Stoffe der Formel (I) für diese Reste als bevorzugt genannt wurden. Hal steht für Chlor oder Brom.

Die Halogenide der Formel (III) sind bekannt oder lassen sich nach bekannten Methoden herstellen.

Als Verdünnungsmittel kommen bei der Durchführung des erfindungsgemäßen Verfahrens alle üblichen inerten, organischen Solventien in Betracht. Vorzugsweise verwendbar sind aliphatische, cycloaliphatische und aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; außerdem Ether, wie Diethylether, Diisopropylether, Dioxan, Tetrahydrofuran oder Ethylenglykoldimethyl- oder - diethylether, ferner Ketone, wie Aceton oder Butanon oder Methyl-isobutyl-keton; Nitrile wie Acetonitril, Propionitril oder Benzonitril, oder Ester wie Essigsäuremethylester oder Essigsäureethylester.

Bei der Durchführung des erfindungsgemäßen Verfahrens arbeitet man vorzugsweise in Gegenwart eines Säurebindemittels. Als solche kommen alle üblichen anorganischen oder organischen Basen infrage. Vorzugsweise verwendbar sind Erdalkali- oder Alkalimetallhydroxide wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen 0 und 150°C, vorzugsweise bei Temperaturen zwischen 20 und 120°C.

Das erfindungsgemäße Verfahren wird üblicherweise unter Normaldruck durchgeführt. Es ist jedoch auch möglich, unter erhöhtem oder vermindertem Druck zu arbeiten.

Zur Durchführung des erfindungsgemäßen Verfahrens setzt man pro Mol an Benzotriazol der Formel (II) in einem Verdünnungsmittel im allgemeinen 1,0 bis 2,0 Mol, vorzugsweise 1,0 bis 1,3 Mol an Halogenid der Formel (III) und gegebenenfalls 1,0 bis 2,0 Mol, vorzugsweise 1,0 bis 1,3 Mol an Säurebindemittel ein. Die Reaktionsführung, Aufarbeitung und Isolierung der Reaktionsprodukte erfolgt nach bekannten Verfahren (vgl. auch die Herstellungsbeispiele).

Die Benzotriazole der Formel (I) können in Säureadditions-Salze oder Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z. B. Ethanol und Hinzufügen zu Verbindungen der Formel (I). Man kann Metallsalz-Komplexe in bekannter Weise, z. B. durch Abfiltrieren isolieren und gegebenenfalls durch Umkristallisation reinigen.

Die erfindungsgemäßen Wirkstoffe weisen eine starke mikrobizide Wirkung auf und können zur Bekämpfung unerwünschter Mikroorganismen, wie Fungi und Bakterien, im Pflanzenschutz und im Materialschutz eingesetzt werden.

Zu den unerwünschten Mikroorganismen gehören Pilze, wie Plasmodiophoromycetes, Oomycetes, Chytridiomycetes, Zygomycetes, Ascomycetes, Basidiomycetes, Deuteromycetes und ferner Bakterien, wie Pseudomonadaceae, Rhizobiaceae, Enterobacteriaceae, Corynebacteriaceae und Streptomycetaceae.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Krankheiten im Obst-, Gemüse- und Weinbau, wie beispielsweise gegen Phytophthora- oder Plasmopara-Arten, oder zur Bekämpfung von Reis-Krankheiten, wie beispielsweise gegen den Erreger der Reisfleckenkrankheit (Pyricularia oryzae) eingesetzt werden.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in übliche Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel wie Alkohole als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

In den Formulierungen können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Pflanzenschutz im allgemeinen zwischen 0,1 und 95 Gewichtsprozent Wirkstoff, vorzugsweise zwischen 0,5 und 90 %.

Die erfindungsgemäßen Wirkstoffe können bei Verwendung im Pflanzenschutz in den Formulierungen in Mischung mit bekannten Fungiziden, Bakteriziden, Akariziden, Nematiziden oder Insektiziden eingesetzt werden, um so z.B. das Wirkungsspektrum zu verbreitern oder Resistenzentwicklungen vorzubeugen.

Für die Mischungen kommen beispielsweise folgende Stoffe in Frage.

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3 -thiazol-5-carboxanilid; 2,6-Dichloro-N-(4-trifluoromethylbenzyl)-benzamid; (E)-2-Methoximino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]-acetat; 2-Phenylphenol (OPP), Aldimorph, Ampropylfos, Anilazin, Azaconazol, Benalaxyl, Benodanil, Benomyl, Binapacryl, Biphenyl, Bitertanol, Blasticidin-S, Bromuconazole, Bupirimate, Buthiobate, Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitröpan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alpha-methrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen, Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0.0001 bis 0,02 Gew.-% am Wirkungsort erforderlich.

Die zum Schutz technischer Materialien verwendeten Mittel enthalten die Wirkstoffe im allgemeinen in einer Menge von 1 bis 95%, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

In vielen Fällen erhält man dabei synergistische Effekte, d.h. die Wirksamkeit der Mischung ist größer als die Wirksamkeit der Einzelkomponenten. Besonders günstige Mischungspartner sind z.B. die folgenden Verbindungen:
Sulfenamide wie Dichlofluanid, Tolylfluanid, Folpet, Fluorfolpet;
Benzimidazole wie Carbendazim (MBC), Benomyl, Fuberidazole, Thiabendazole oder deren Salze;
Thiocyanate wie Thiocyanatomethylthiobenzothiazol (TCMTB), Methylenbisthiocyanat (MBT);
quartäre Ammoniumverbindungen wie Benzyldimethyltetradecylammoniumchlorid, Benzyl-dimethyl-dodecyl-ammoniumchlorid, Dodecyl-dimethyl-ammoniumchlorid;
Morpholinderivate wie C₁₁-C₁₄-4-Alkyl-2,6-dimethyl-morpholinhomologe (Tridemorph), (±)-cis-4-[3-(4-tert.-Butylphenyl)-2-methylpropyl]-2,6-dimethylmorpholin (Fenpropimorph), Falimorph;
Phenole wie o-Phenylphenol, Tribromphenol, Tetrachlorphenol, Pentachlorphenol, 3-Methyl-4-chlorphenol, Dichlorophen, Chlorophen oder deren Salze;
Azole wie Triadimefon, Triadimenol, Bitertanol, Tebuconazole, Propiconazole, Azaconazole, Hexaconazole, Prochloraz, Cyproconazole, 1-(2-Chlorphenyl)-2-(1-chlorcyclopropyl)-3-(1,2,4-triazol-1-yl)-propan-2-ol oder 1-(2-Chlorphenyl)-2-(1,2,4-triazol-1-yl-methyl)-3,3-dimethyl-butan-2-ol.
Iodpropargylderivate wie Iodpropargyl-butylcarbamat (IPBC), -chlorophenylformal, -phenylcarbamat, -hexylcarbamat, -cyclohexylcarbamat, Iodpropargyloxyethylphenylcarbamat;
Iodderivate wie Diiodmethyl-p-arylsulfone z.B. Diiodmethyl-p-tolylsulfon; Bromderivate wie Bromopol;
Isothiazoline wie N-Methylisothiazolin-3-on, 5-Chloro-N-methylisothiazolin-3-on, 4,5-Dichlor-N-octylisothiazolin-3-on, N-Octylisothiazolin-3-on (Octilinone);
Benzisothiazolinone, Cyclopentenisothazoline;
   Pyridine wie 1-Hydroxy-2-pyridinthion (und ihre Na-, Fe-, Mn, Zn-Salze), Tetrachlor-4-methylsulfonylpyridin;
Metallseifen wie Zinn-, Kupfer-, Zink-napthenat, -octoat, -2-ethylhexanoat, -oleat,
-phosphat, -benzoat, Oxide wie TBTO, Cu₂O, CuO, ZnO;
Organische Zinnverbindungen wie Tributylzinnnaphtenat und Tributylzinnoxid;
Dialkyldithiocarbamate wie Na- und Zn-Salze von Dialkyldithiocarbamaten, Tetramethylthiuramdisulfid (TMTD);
Nitrile wie 2,4,5,6-Tetrachlorisophthalonitril (Chlorthalonil) u.a. Mikrobizide mit aktivierter Halogengruppe wie Cl-Ac, MCA, Tectamer, Bromopol, Bromidox;
Benzthiazole wie 2-Mercaptobenzothiazole; s.o. Dazomet;
Chinoline wie 8-Hydroxychinolin;
Formaldehydabspaltende Verbindungen wie Benzylalkoholmono(poly)hemiformal, Oxazolidine, Hexahydro-s-triazine, N-Methylolchloracetamid;
Tris-N-(Cyclohexyldiazeniumdioxy)-Aluminium, N-(Cyclohexyldiazeniumdioxy)-Tributylzinn bzw. K-Salze, Bis-(N-cyclohexyl)diazinium -(dioxy-Kupfer oder Aluminium).

### Als Insektizide werden bevorzugt zugesetzt:

Phosphorsäureester wie Azinphos-ethyl, Azinphos-methyl, 1-(4-Chlorphenyl)-4-(O-ethyl, S-propyl)phosphoryloxypyrazol (TIA-230), Chlorpyrifos, Coumaphos, Demeton, Demeton-S-methyl, Diazinon, Dichlorfos, Dimethoate, Ethoprophos, Etrimfos, Fenitrothion, Fenthion, Heptenophos, Parathion, Parathion-methyl, Phosalone, Phoxim, Pirimiphos-ethyl, Pirimiphos-methyl, Profenofos, Prothiofos, Sulprofos, Triazophos und Trichlorphon.

Carbamate wie Aldicarb, Bendiocarb, BPMC (2-(1-Methylpropyl)phenylmethylcarbamat), Butocarboxim, Butoxycarboxim, Carbaryl, Carbofuran, Carbosulfan, Cloethocarb, Isoprocarb, Methomyl, Oxamyl, Pirimicarb, Promecarb, Propoxur und Thiodicarb.

Pyrethroide wie Allethrin, Alphamethrin, Bioresmethrin, Bifenthrin (FMC 54800), Cycloprothrin, Cyfluthrin, Decamethrin, Cyhalothrin, Cypermethrin, Deltamethrin, Alpha-cyano-3-phenyl-2-methylbenzyl-2,2-dimethyl-3-(2-chlor-2-trifluormethylvinyl)cyclopropancarboxylat, Fenpropathrin, Fenfluthrin, Fenvalerate, Flucythrinate, Flumethrin, Fluvalinate, Permethrin und Resmethrin; Nitroimino- und Nitromethylen-Verbindungen wie 1-[(6-Chlor-3-pyridinyl)-methyl]-4,5-dihydro-N-nitro-lH-imidazol-2-amin (Imidacloprid).

Organosiliciumverbindungen, vorzugsweise Dimethyl(phenyl)silylmethyl-3-phenoxybenzylether wie z.B. Dimethyl-(4-ethoxyphenyl)-silylmethyl-3-phenoxybenzylether oder Dimethyl(phenyl)-silylmethyl-2-phenoxy-6-pyridylmethylether wie z.B. Dimethyl(9-ethoxyphenyl)-silylmethyl-2-phenoxy-6-pyridylmethylether oder (Phenyl)[3-(3-phenoxyphenyl)propyl](dimethyl)-silane wie z.B. (4-Ethoxyphenyl)-[3(4-fluoro-3-phenoxyphenyl)-propyl]dimethylsilan.

Als andere Wirkstoffe kommen in Betracht Algizide, Molluskizide, Wirkstoffe gegen "sea animals", die sich auf z.B. Schiffsbodenanstrichen ansiedeln.

Die Herstellung und die Verwendung von erfindungsgemäßen Wirkstoffen werden durch die folgenden Beispiele veranschaulicht.

### Herstellungsbeispiele:

### Beispiel 1

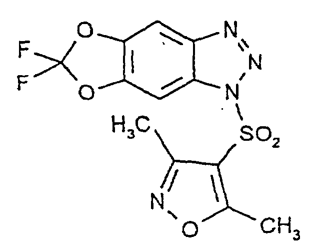

Ein Gemisch aus 2,0 g (10 mmol) 6,6-Difluor-[1,3]dioxolo[4,5-f]-1H-benzotriazol und 40 ml absolutem Tetrahydrofuran wird bei Raumtemperatur unter Rühren mit 0,3 g (10 mmol) Natriumhydrid (80%ig) versetzt und danach 10 Minuten bei Raumtemperatur gerührt. Anschließend fügt man 1,9 g (10 mmol)-3,5-Dimethylisoxazol-4-sulfonylchlorid hinzu und rührt 16 Stunden bei 60°C. Zur Aufarbeitung wird das Reaktionsgemisch in 20 ml Wasser gegossen. Das entstehende Gemisch wird zweimal mit je 50 ml Essigsäureethylester extrahiert. Die vereinigten organischen Phasen werden über Natriumsulfat getrocknet und unter vermindertem Druck eingeengt. Der verbleibende Rückstand wird mit Methylenchlorid als Laufmittel an Kieselgel chromatographiert. Man erhält auf diese Weise 2,7 g (75% der Theorie) an 1-(3,5-Dimethylisoxazol-4-sulfonyl)-6,6-difluor-[1,3]dioxolo[4,5-f]-benzotriazol in Form eines farblosen Feststoffes vom Schmelzpunkt 120-123°C.

### Beispiel 2 :

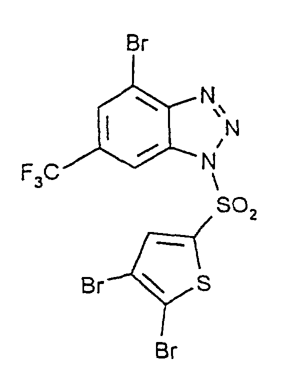

Ein Gemisch aus 2,7 g (10 mmol) 4-Brom-6-trifluormethyl-1H-benzotriazol und 40 ml absolutem Tetrahydrofuran wird bei Raumtemperatur unter Rühren mit 0,3 g (10 mmol) Natriumhydrid (80%ig) versetzt und danach 10 Minuten bei Raumtemperatur gerührt. Anschließend fügt man 3,4 g (10 mmol) 4,5-Dibromthiophen-2-sulfonylchlorid hinzu und rührt 16 Stunden bei 60°C. Zur Aufarbeitung wird das Reaktionsgemisch in 200 ml Wasser gegeben. Das entstehende Gemisch wird zweimal mit je 50 ml Essigsäureethylester extrahiert. Die vereinigten organischen Phasen werden über Natriumsulfat getrocknet und unter vermindertem Druck eingeengt. Der verbleibende Rückstand wird mit Methylenchlorid als Laufmittel an Kieselgel chromatographiert. Man erhält auf diese Weise 2,8 g (52% der Theorie) an 1-(3,4-Dibrom-thien-2-yl-sulfonyl)-4-brom-6-trifluormethyl-1H-benzotriazol in Form eines farblosen Feststoffes vom Schmelzpunkt 192-195°C.

Nach den zuvor angegebenen Methoden werden auch die in der folgenden Tabelle 9 aufgeführten Stoffe der Formel 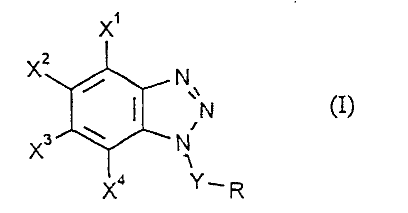 hergestellt.

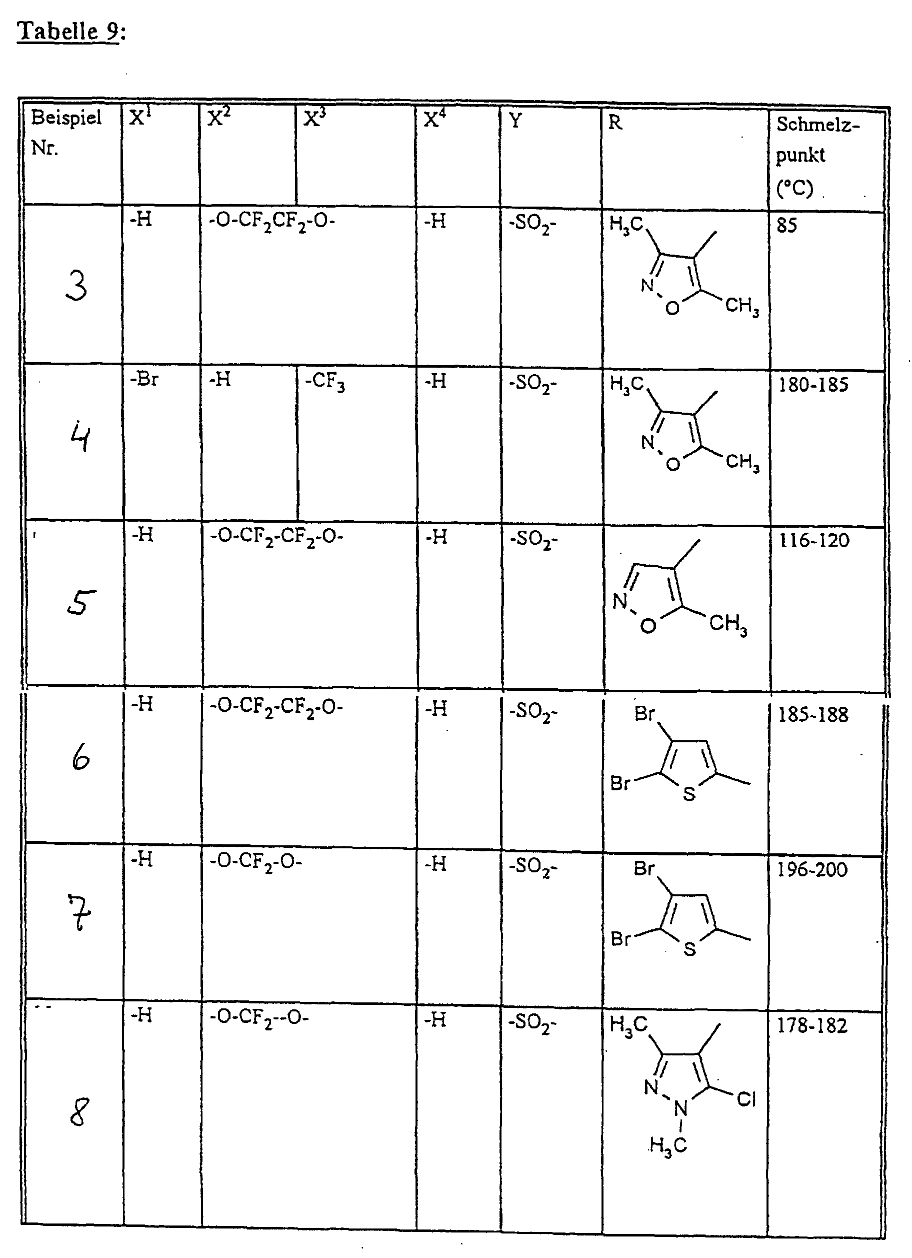

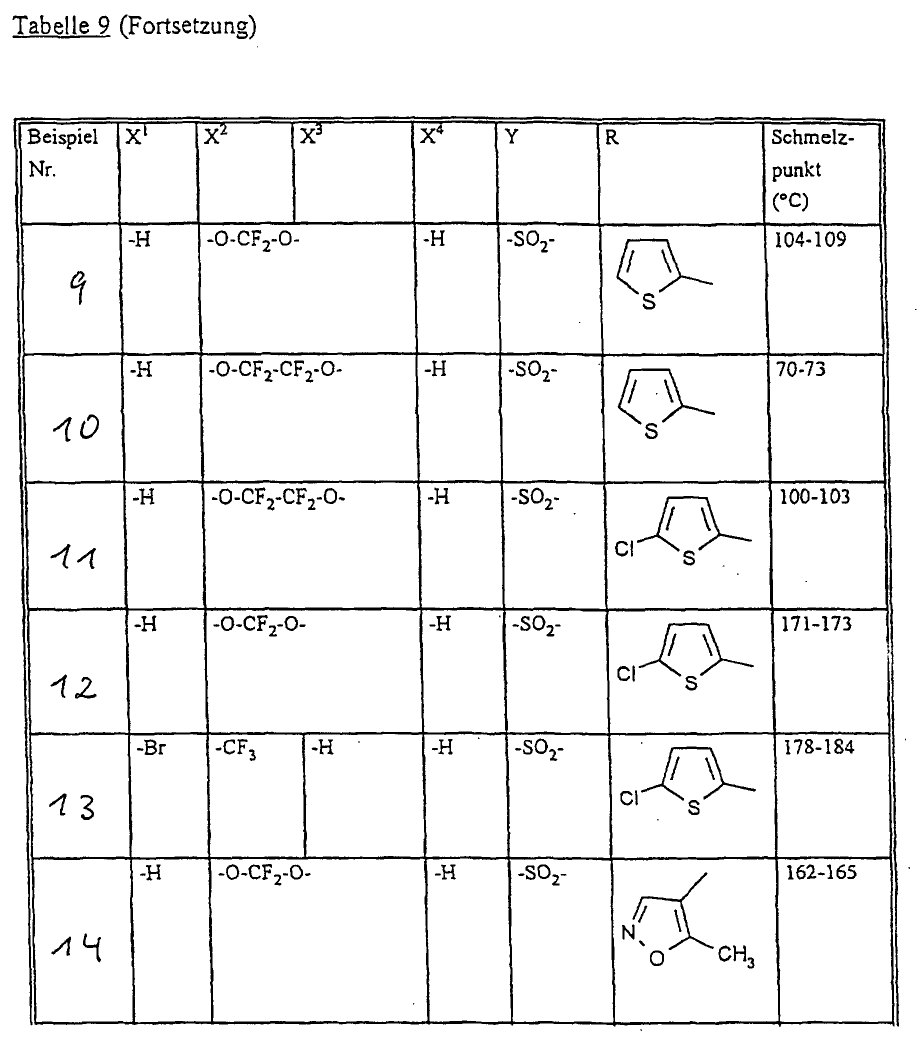

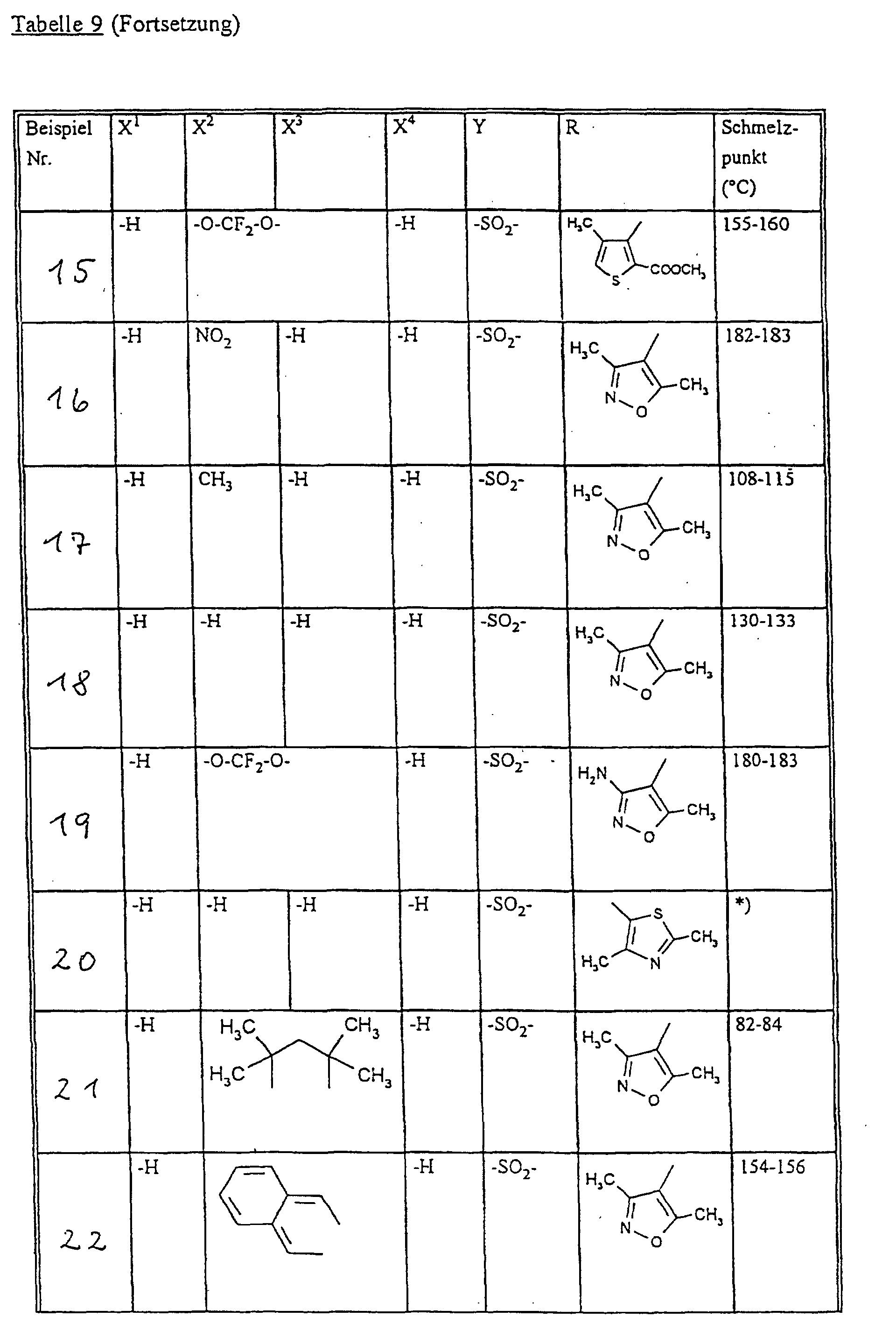

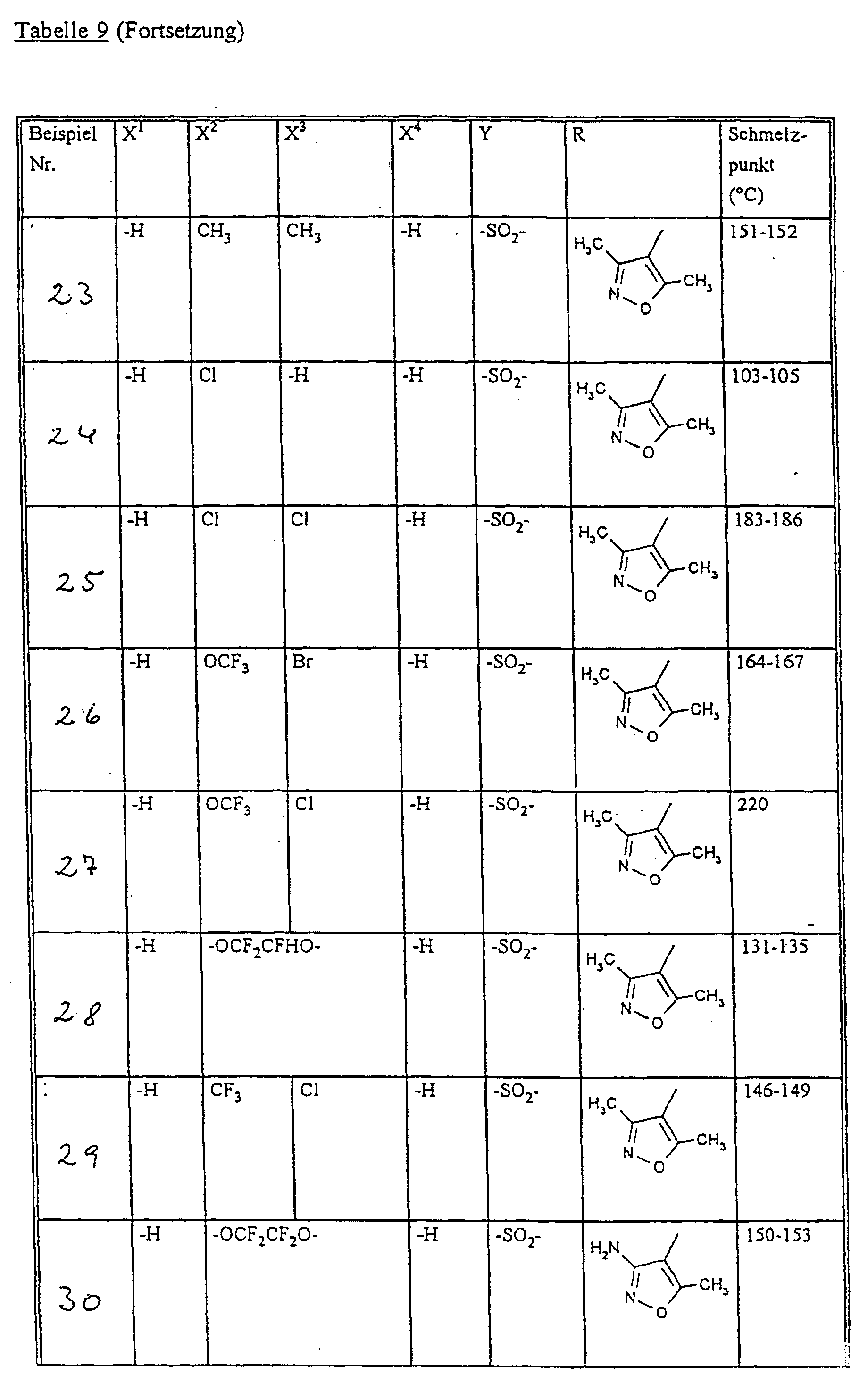

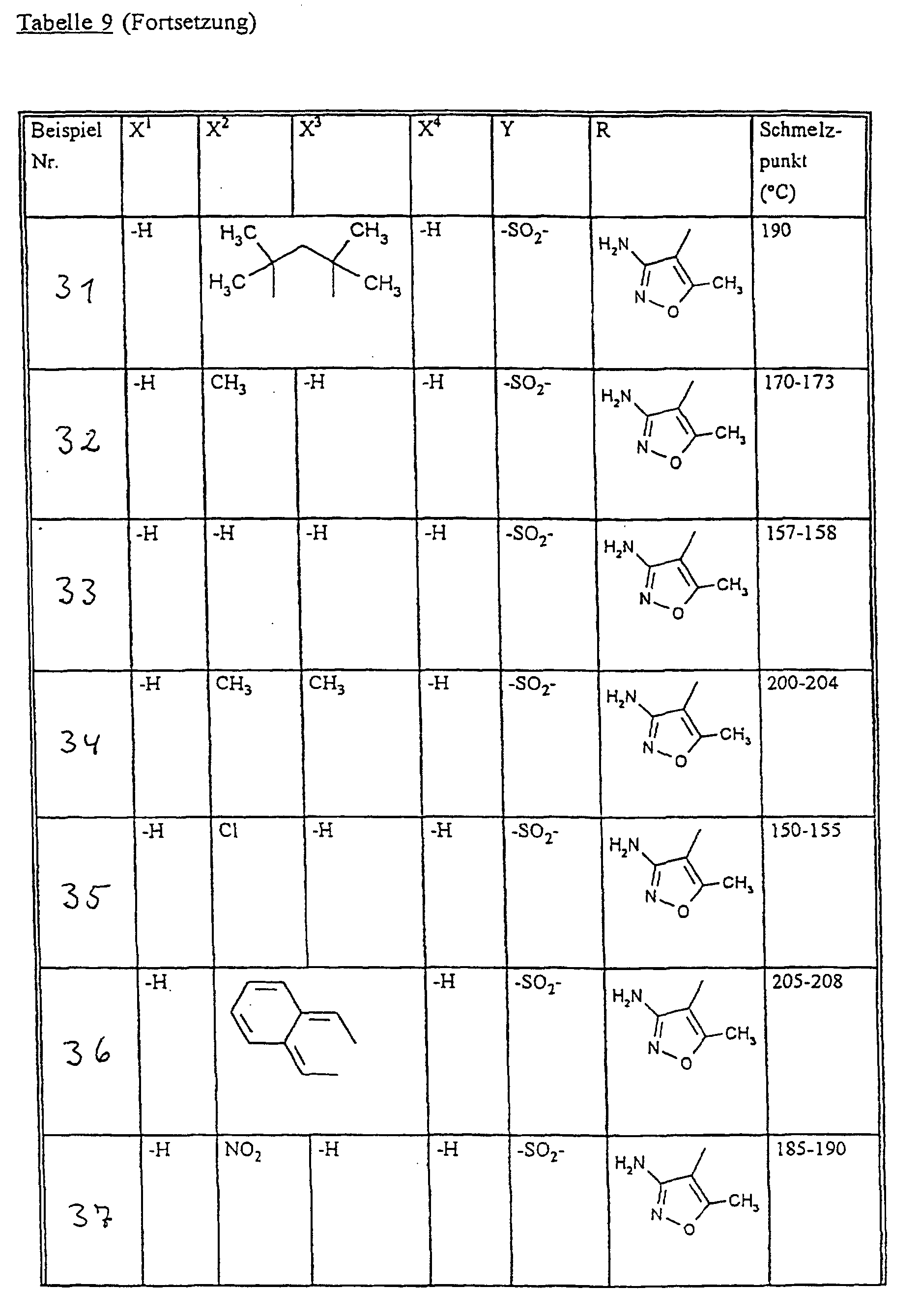

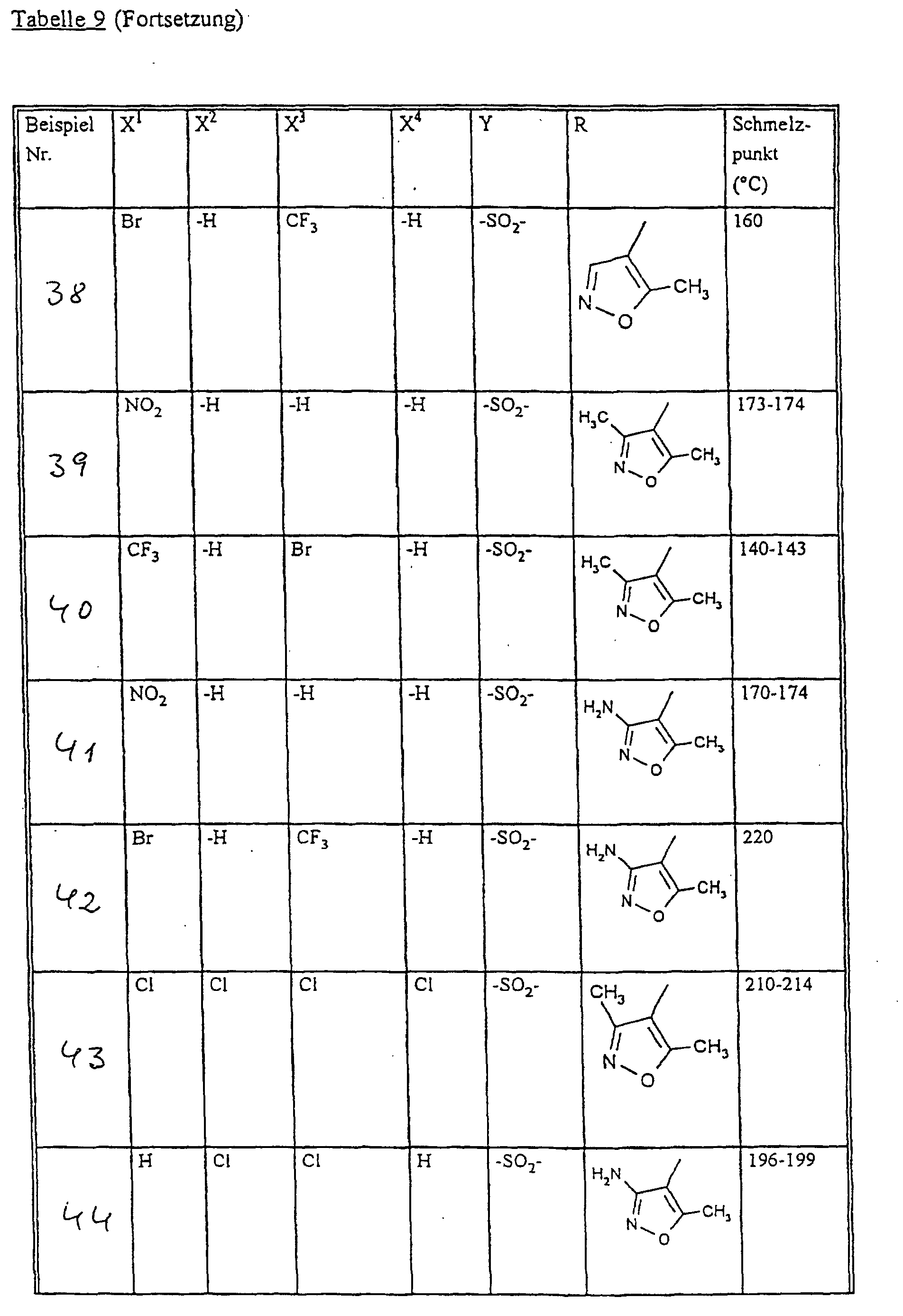

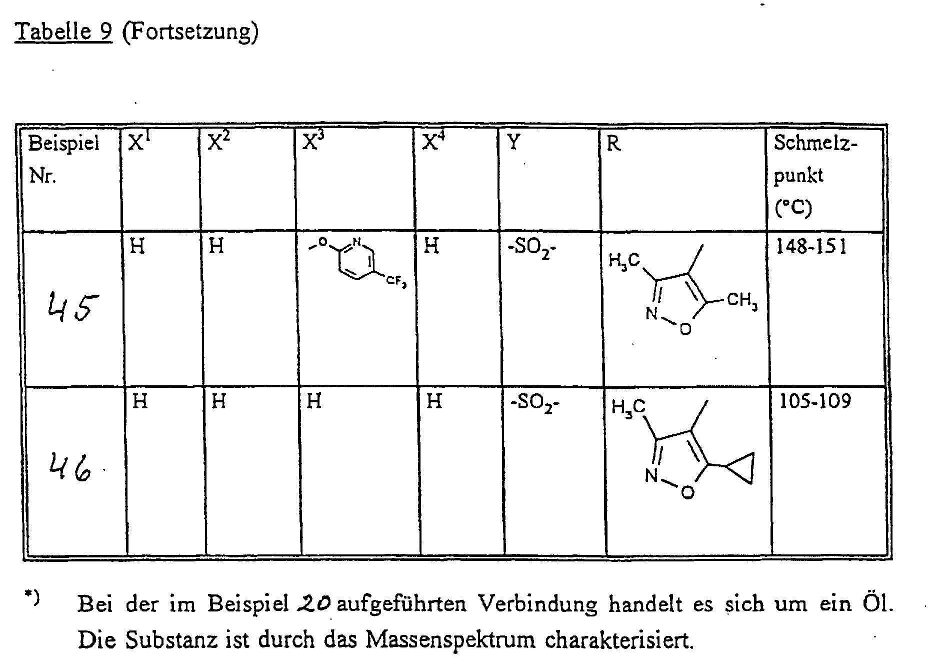

MS: m/Z
294, 225, 201, 176, 135, 112, 106, 71, 45.

### Verwendungsbeispiele:

### Beispiel A

### Materialschutz-Test

Zum Nachweis der Wirksamkeit gegen Pilze werden die minimalen Hemmkonzentrationen (MKH-Werte) von erfindungsgemäßen Verbindungen bestimmt:

Ein Agar, der unter Verwendung von Malzextrakt hergestellt wird, wird mit erfindungsgemäßen Wirkstoffen in Konzentrationen von 0,1 mg/l bis 5.000 mg/l versetzt. Nach Erstarren des Agars erfolgt Kontamination mit Reinkulturen von Penicillium brevicaule, Chaetomium globosum und Aspergillus niger. Nach zweiwöchiger Lagerung bei 28°C und 60 bis 70 % relativer Luftfeuchtigkeit wird die minimale Hemmkonzentration (MKH-Wert) bestimmt. Der MKH-Wert kennzeichnet die niedrigste Konzentration an Wirkstoff, bei der keinerlei Bewuchs durch die verwendete Mikrobenart erfolgt.

Wirkstoffe und Versuchsergebnisse gehen aus der folgenden Tabelle hervor.

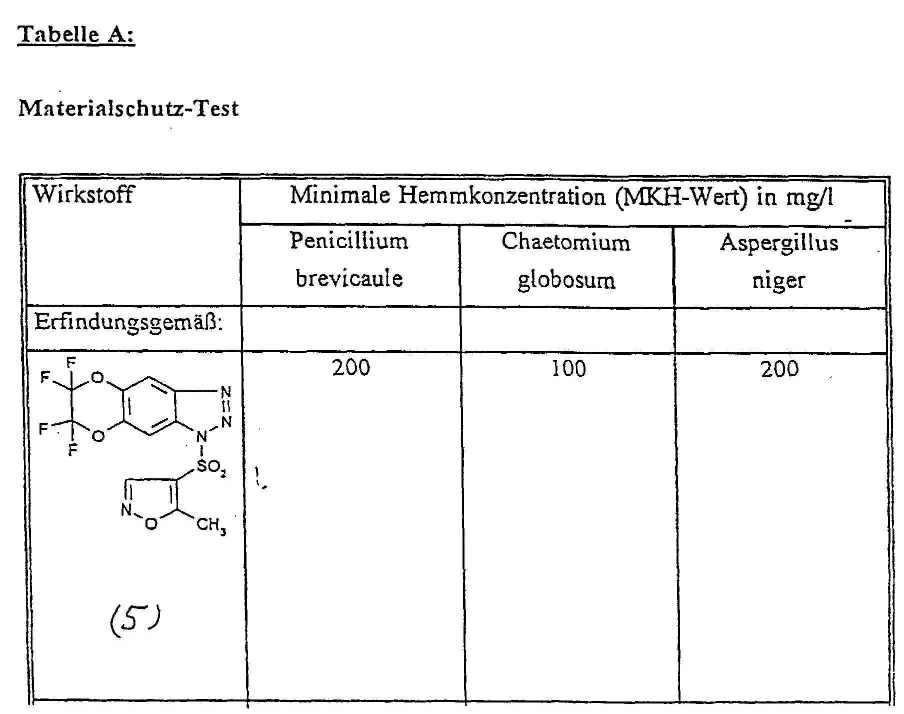

### Beispiel B:

### Phytophthora-Test (Tomate) / protektiv

| | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen mit einer wäßrigen Sporensuspension von *Phytophthora infestans* inokuliert.

Die Pflanzen werden in einer Inkubationskabine mit 100% relativer Luftfeuchtigkeit und ca. 20°C aufgestellt.

3 Tage nach der Inokulation erfolgt die Auswertung. Dabei bedeutet 0 % einen Wirkungsgrad der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus den folgenden Tabellen hervor.

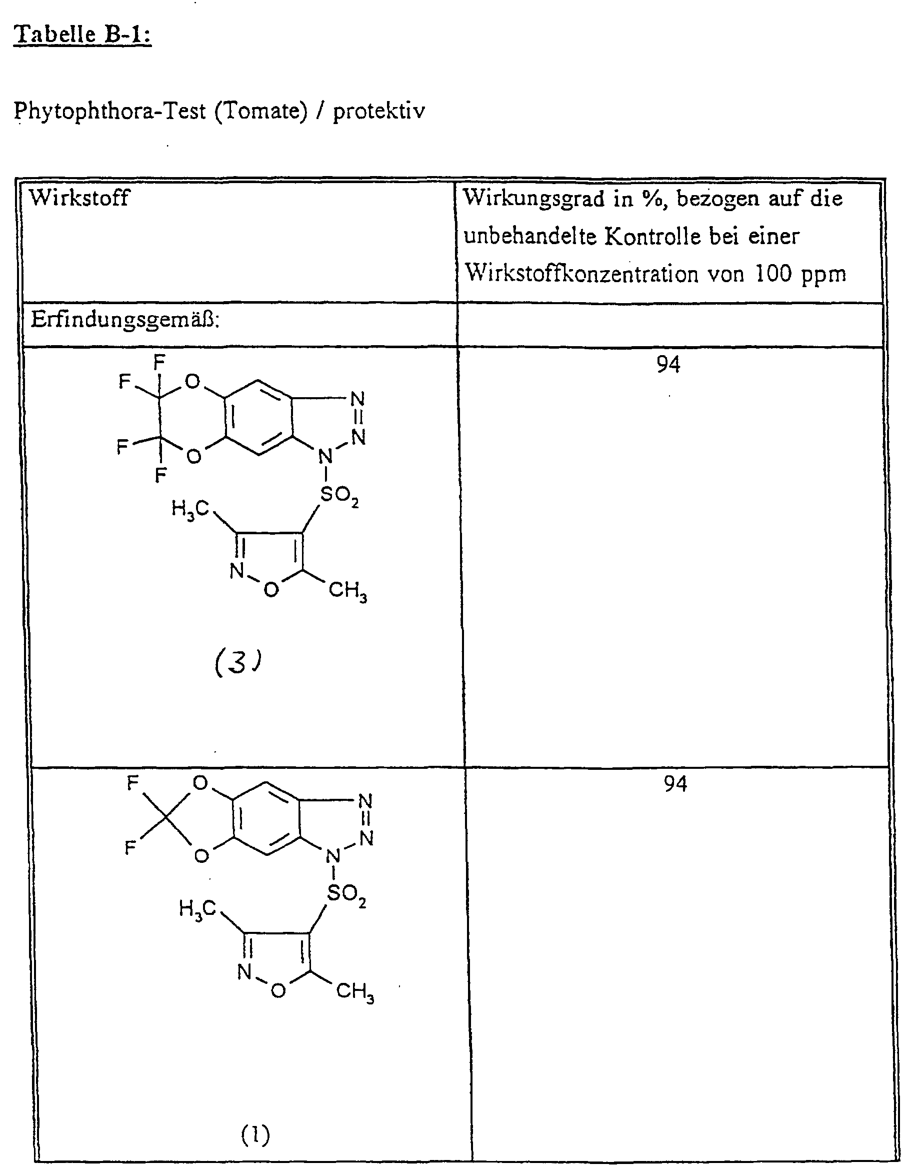

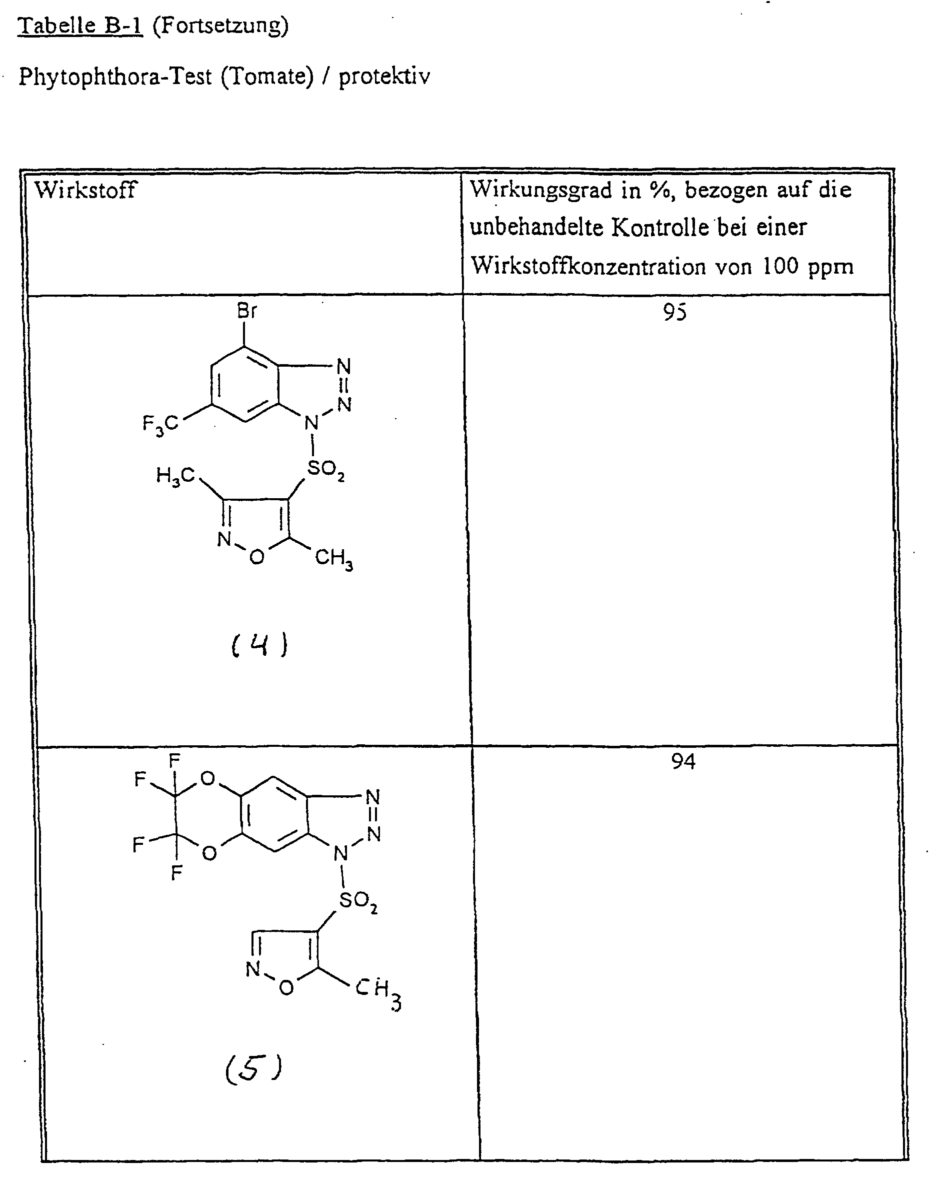

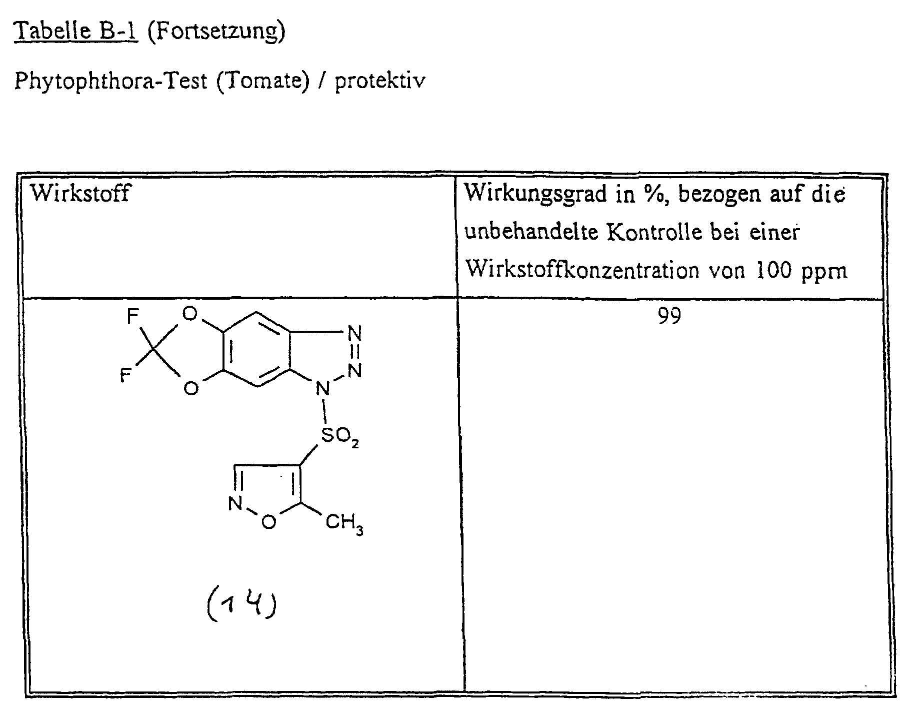

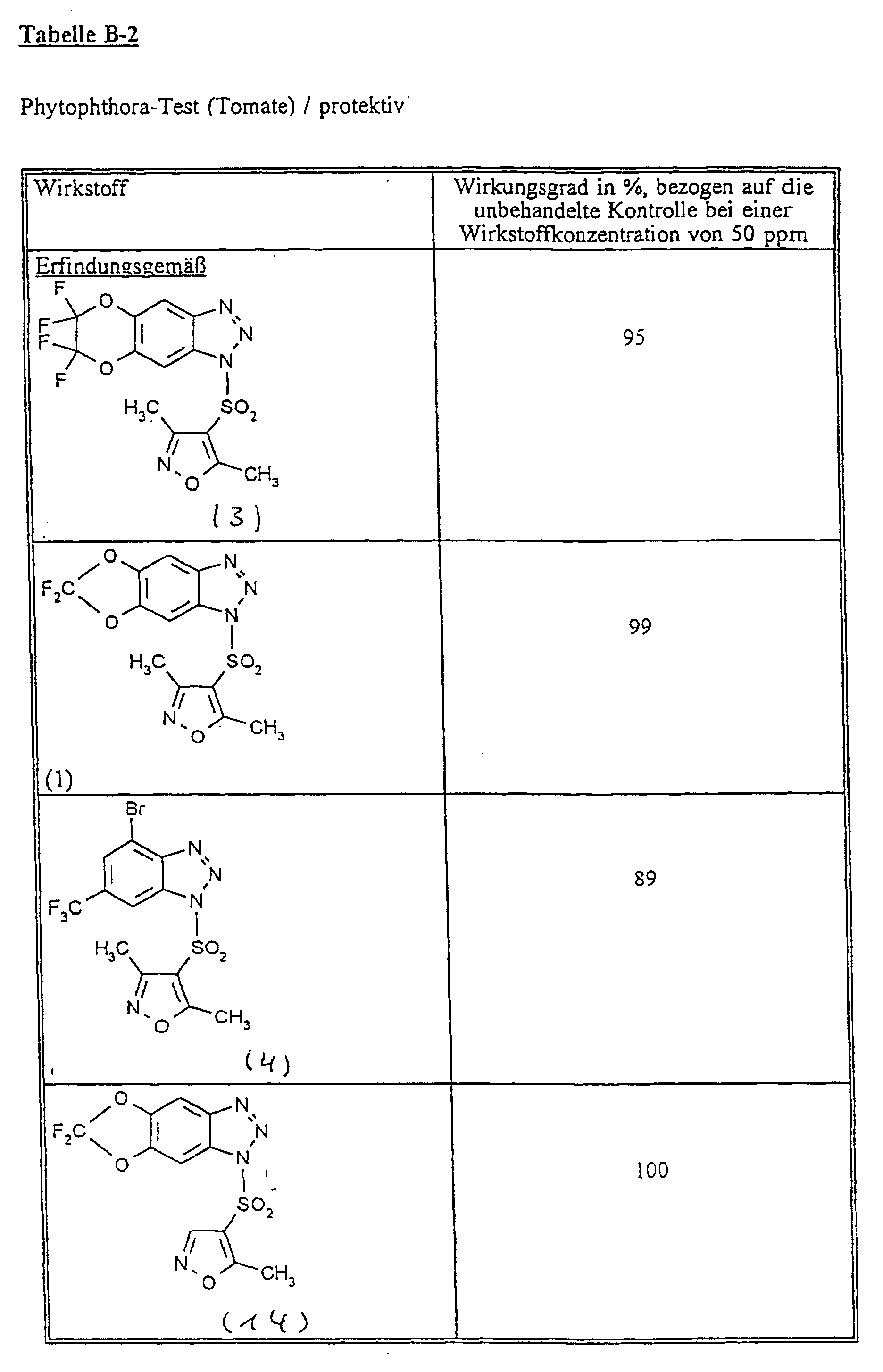

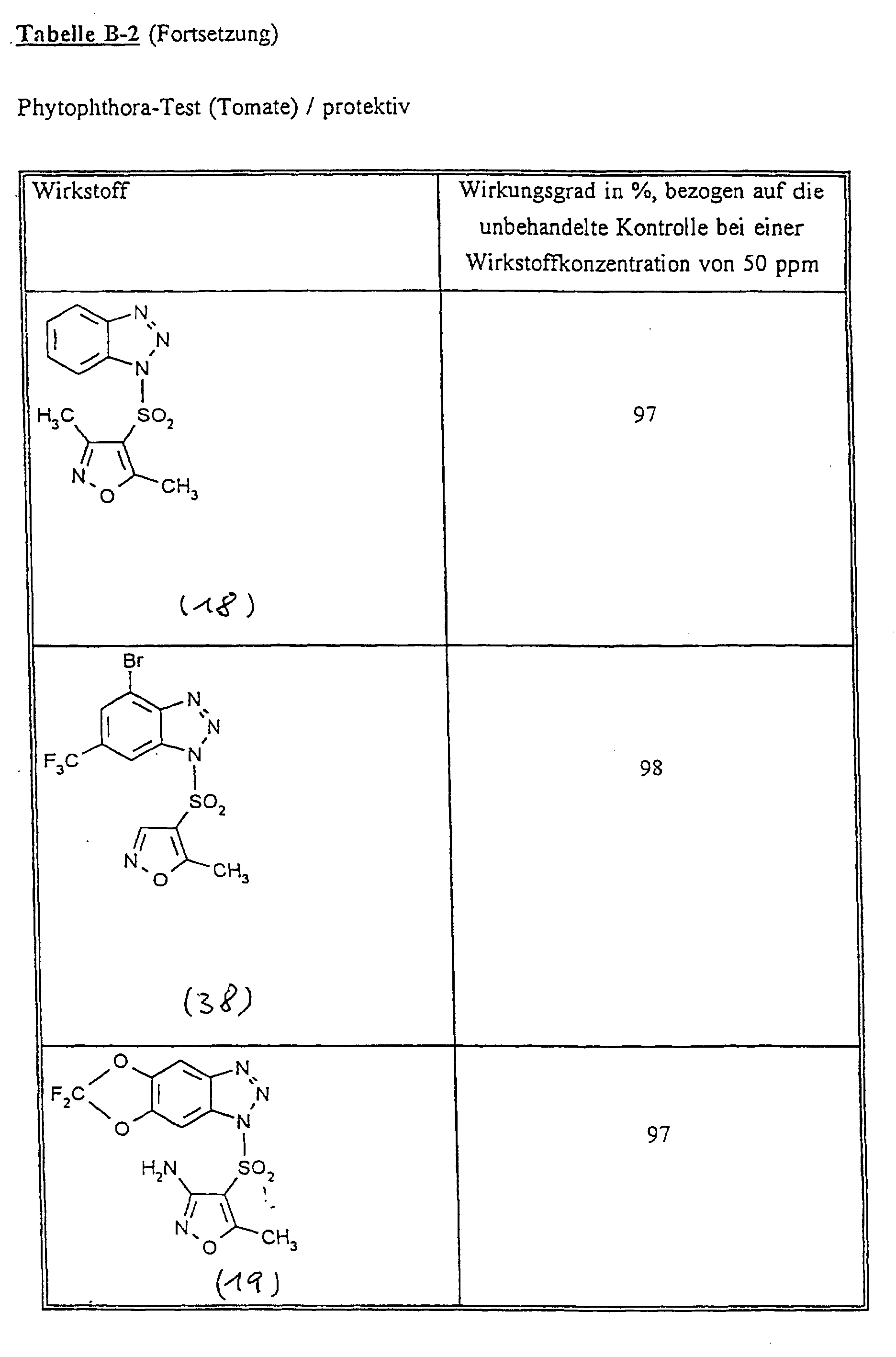

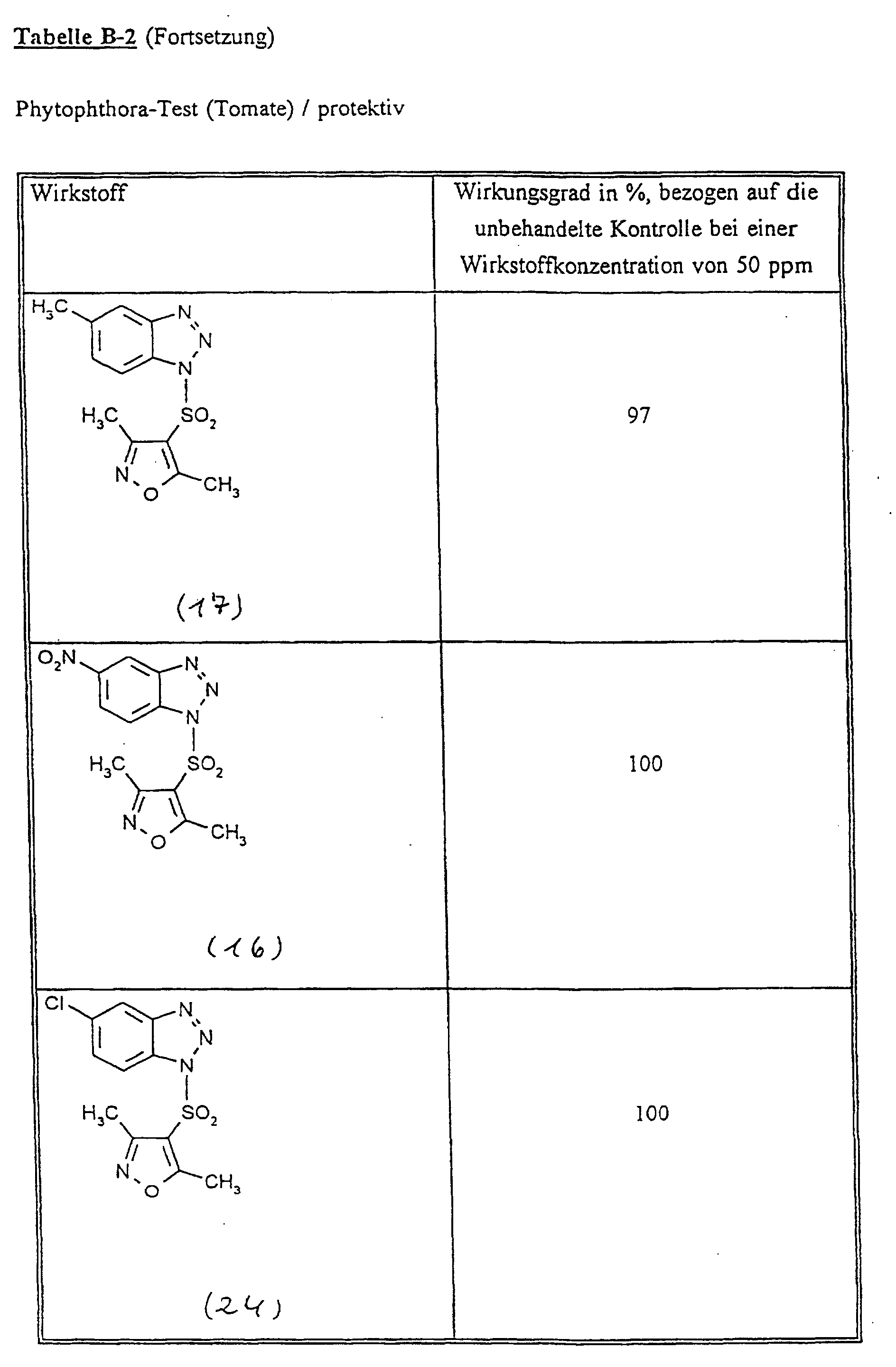

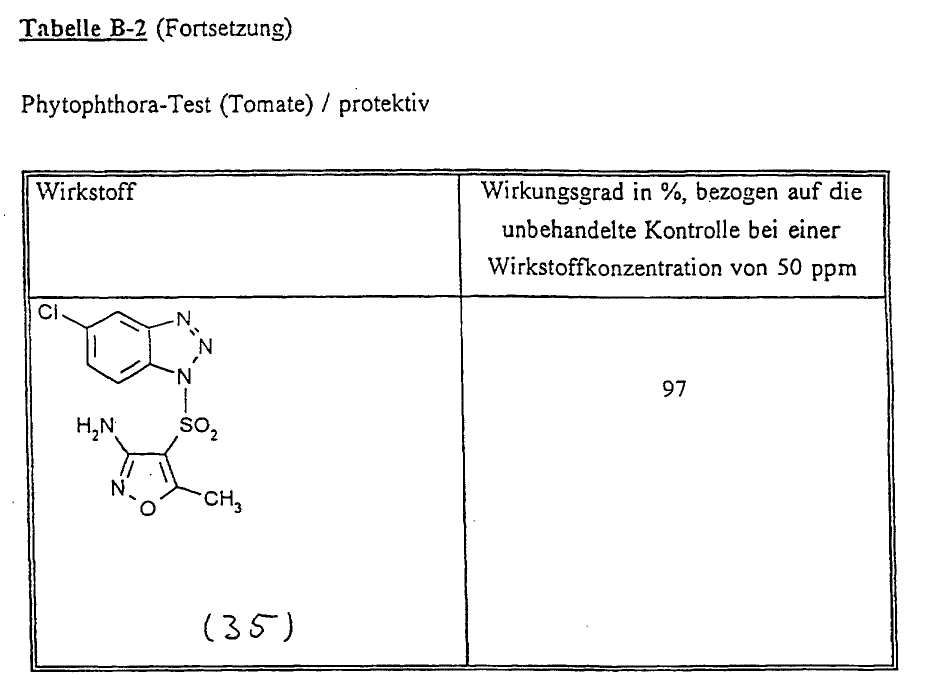

### Beispiel C:

### Plasmopara-Test (Reben) / protektiv

| | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100 % relativer Luftfeuchtigkeit. Anschließend werden die Pflanzen 5 Tage im Gewächshaus bei 21°C und ca. 90 % Luftfeuchtigkeit aufgestellt. Die Pflanzen werden dann angefeuchtet und 1 Tag in eine Feuchtkammer gestellt.

6 Tage nach der Inokulation erfolgt die Auswertung. Dabei bedeutet 0 % einen Wirkungsgrad, der demjenigen der unbehandelten Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

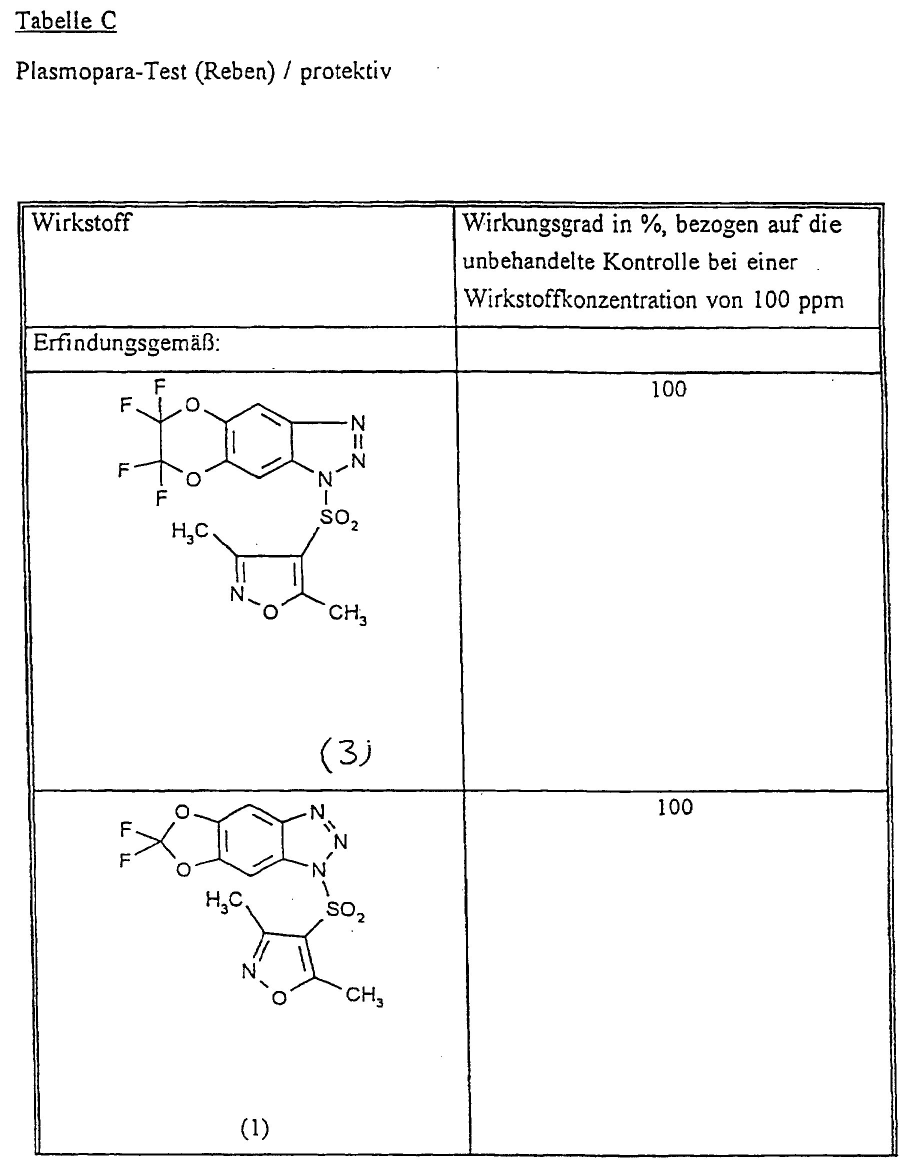

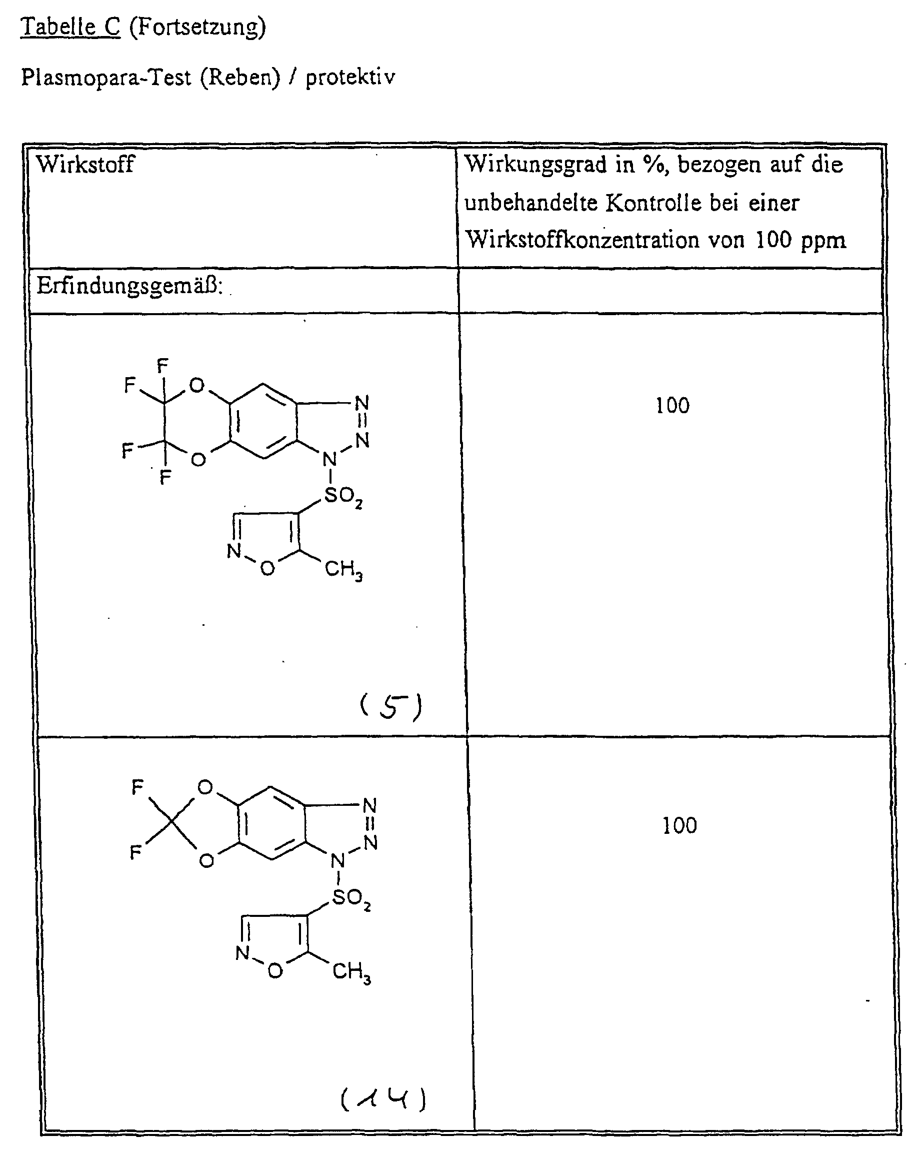

## Patentansprüche

1. Benzotriazole der Formel 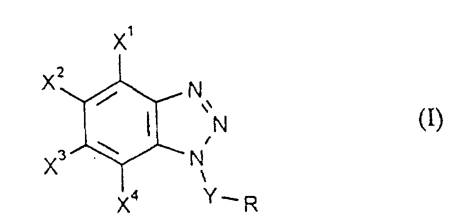 in welcher
X¹, X², X³ und X⁴ unabhängig voneinander für Wasserstoff, Fluor, Chlor, Brom, Iod, Cyano, Nitro, geradkettiges oder verzweigtes Alkyl mit 1 bis 8 Kohlenstoffatomen, geradkettiges oder verzweigtes Halogenalkyl mit 1 bis 6 Kohlenstoffatomen und 1 bis 13 gleichen oder verschiedenen Halogenatomen, geradkettiges oder verzweigtes Alkoxy mit 1 bis 8 Kohlenstoffatomen, geradkettiges oder verzweigtes Halogenalkoxy mit 1 bis 6 Kohlenstoffatomen und 1 bis 13 gleichen oder verschiedenen Halogenatomen, geradkettiges oder verzweigtes Alkylthio mit 1 bis 8 Kohlenstoffatomen, geradkettiges oder verzweigtes Halogenalkylthio mit 1 bis 6 Kohlenstoffatomen und 1 bis 13 gleichen oder verschiedenen Halogenatomen, geradkettiges oder verzweigtes Alkylsulfinyl mit 1 bis 8 Kohlenstoffatomen, geradkettiges oder verzweigtes Halogenalkylsulfinyl mit 1 bis 6 Kohlenstoffatomen und 1 bis 13 gleichen oder verschiedenen Halogenatomen, geradkettiges oder verzweigtes Alkylsulfonyl mit 1 bis 8 Kohlenstoffatomen, geradkettiges oder verzweigtes Halogenalkylsulfonyl mit 1 bis 6 Kohlenstoffatomen und 1 bis 13 gleichen oder verschiedenen Halogenatomen, gegebenenfalls einfach bis fünffach, gleichartig oder verschieden durch Halogen und/oder Alkyl mit 1 bis 4 Kohlenstoffatomen substituiertes Cycloalkyl mit 3 bis 6 Kohlenstoffatomen, für Hydroxycarbonyl, Alkylcarbonyl mit 1 bis 6 Kohlenstoffatomen im geradkettigen oder verzweigten Alkylteil, Alkoxycarbonyl mit 1 bis 6 Kohlenstoffatomen im geradkettigen oder verzweigten Alkoxyteil, Cycloalkylcarbonyl mit 3 bis 6 Kohlenstoffatomen im Cycloalkylteil. Cycloalkoxycarbonyl mit 3 bis 6 Kohlenstoffatomen im Cycloalkylteil, für 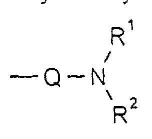 oder -Z-R³ stehen,
worin
R¹ und R² unabhängig voneinander für Wasserstoff, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, geradkettiges oder verzweigtes Alkoxyalkyl mit 1 bis 4 Kohlenstoffatomen im Alkoxyteil und 1 bis 4 Kohlenstoffatomen im Alkylteil, Alkylcarbonyl mit 1 bis 6 Kohlenstoffatomen im geradkettigen oder verzweigten Alkylteil, Aryl mit 6 bis 10 Kohlenstoffatomen, Arylcarbonyl mit 6 bis 10 Kohlenstoffatomen im Arylteil stehen, Arylsulfonyl mit 6 bis 10 Kohlenstoffatomen, Arylaminocarbonyl mit 6 bis 10 Kohlenstoffatomen im Arylteil oder für Arylmethylsulfonyl mit 6 bis 10 Kohlenstoffatomen im Arylteil stehen, wobei jeder der zuvor genannten Aryl-Reste einfach bis dreifach, gleichartig oder verschieden substituiert sein kann durch Halogen, Cyano, Nitro, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Halogenalkylthio mit 1 bis 4 Kohlenstoffatomen und 1 bis 5 gleichen oder verschiedenen Halogenatomen, Alkylsulfinyl mit 1 bis 4 Kohlenstoffatomen, Alkylsulfonyl mit -1 bis 4 Kohlenstoffatomen, Halogenalkylsulfinyl mit 1 bis 4 Kohlenstoffatomen und 1 bis 5 gleichen oder verschiedenen Halogenatomen und/oder Halogenalkylsulfonyl mit 1 bis 4 Kohlenstoffatomen und 1 bis 5 gleichen oder verschiedenen Halogenatomen,
oder
R¹ und R² auch gemeinsam mit dem Stickstoffatom, an das sie gebunden sind, vorzugsweise für einen gegebenenfalls einfach bis dreifach durch Alkyl mit 1 bis 4 Kohlenstoffatomen substituierten heterocyclischen Ring mit 5 oder 6 Ringgliedern stehen, der noch ein Sauerstoffatom oder eine C₁-C₄-Alkyliminogruppe enthalten kann,
Q für eine direkte Bindung oder für eine Carbonyl-Gruppe steht und
R³ für Aryl mit 6 bis 10 Kohlenstoffatomen steht, wobei jeder dieser Reste einfach bis dreifach, gleichartig oder verschieden substituiert sein kann durch Halogen, Cyano, Nitro, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Halogenalkylthio mit 1 bis 4 Kohlenstoffatomen und 1 bis 5 gleichen oder verschiedenen Halogenatomen, Alkylsulfinyl mit 1 bis 4 Kohlenstoffatomen, Alkylsulfonyl mit 1 bis 4 Kohlenstoffatomen, Halogenalkylsulfinyl mit 1 bis 4 Kohlenstoffatomen und 1 bis 5 gleichen oder verschiedenen Halogenatomen und oder Halogenalkylsulfonyl mit 1 bis 4 Kohlenstoffatomen und 1 bis 5 gleichen oder verschiedenen Halogenatomen, oder
R³ für einen gesättigten oder ungesättigten Heterocyclyl-Rest mit 5 oder 6 Ringgliedern und 1 bis 3 Heteroatomen steht, wobei diese Reste einfach bis dreifach, gleichartig oder verschieden substituiert sein können durch Halogen, Alkyl mit 1 bis 4 Kohlenstoffatomen. Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Alkoxycarbonyl mit 1 bis 3 Kohlenstoffatomen im Alkoxyteil, Cycloalkyl mit 3 bis 6 Kohlenstoffatomen, Cyano und/oder Nitro,
Z für eine direkte Bindung sowie für CH₂, O, S, SO, SO₂, CO, oder
für -CO-O- steht, wobei das Sauerstoffatom mit dem Aryl- bzw. Heterocyclyl-Rest verbunden ist, oder
für -SO₂-O- steht, wobei das Schwefelatom mit dem Aryl- bzw Heterocyclyl-Rest verbunden ist, oder
für -S-CH₂-SO₂- steht, wobei das Schwefelatom der Thio-Gruppe mit dem Aryl- bzw. Heterocyclyl-Rest verbunden ist,
oder
X² und X³ gemeinsam für eine gegebenenfalls einfach bis sechsfach durch Halogen, Alkyl mit 1 bis 4 Kohlenstoffatomen und/oder Halogenalkyl mit 1 bis 4 Kohlenstoffatomen und 1 bis 9 Halogenatomen substituierte Alkylenkette mit 3 oder 4 Gliedern stehen, in der ein oder zwei (nicht benachbarte) Kohlenstoffatome durch Sauerstoffatome ersetzt sein können, oder
X² und X³ gemeinsam für einen Rest der Formel 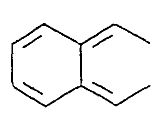 stehen,
R für einen gesättigten oder ungesättigten, Heterocyclyl-Rest mit 5 oder 6 Ringgliedern und 1 bis 3 Heteroatomen steht, wobei diese Reste einfach bis dreifach, gleichartig oder verschieden substituiert sein können durch Halogen, Cyano, Nitro, Hydroxy, Amino, Formyl, Carboxy, Carbamoyl, Thiocarbamoyl, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Alkoxycarbonyl mit 1 bis 4 Kohlenstoffatomen im Alkoxyteil, Cycloalkyl mit 3 bis 6 Kohlenstoffatomen, Halogenalkylsulfinyl mit 1 bis 4 Kohlenstoffatomen und 1 bis 5 gleichen oder verschiedenen Halogenatomen, Halogenalkylsulfonyl mit 1 bis 4 Kohlenstoffatomen und 1 bis 5 gleichen oder verschiedenen Halogenatomen, Alkylamino mit 1 bis 4 Kohlenstoffatomen, Hydroxyalkylamino mit 1 bis 4 Kohlenstoffatomen, Dialkylamino mit 1 bis 4 Kohlenstoffatomen in jeder Alkylgruppe, Alkylcarbonyl mit 1 bis 4 Kohlenstoffatomen im Alkylteil, Alkylcarbonylamino mit 1 bis 4 Kohlenstoffatomen im Alkylteil, Hydroxyiminoalkyl mit 1 bis 4 Kohlenstoffatomen im Alkylteil, Alkoximinoalkyl mit 1 bis 4 Kohlenstoffatomen im Alkoxyteil und 1 bis 4 Kohlenstoffatomen im Alkylteil, Alkylcarbonyloxy mit 1 bis 4 Kohlenstoffatomen im Alkylteil und/oder Halogenalkylcarbonyloxy mit 1 bis 4 Kohlenstoffatomen in der Halogenalkylgruppe und 1 bis 5 gleichen oder verschiedenen Halogenatomen, und wobei die Heterocyclyl-Reste auch Oxo-Gruppen enthalten können,
und
Y für -SO₂- steht,
sowie deren Säureadditions-Salze und Metallsalz-Komplexe.

2. Verfahren zur Herstellung von Benzotriazolen der Formel (I) gemäß Anspruch 1 sowie von deren Säureadditions-Salzen und Metallsalz-Komplexen, **dadurch gekennzeichnet, daß** man Benzotriazole der Formel 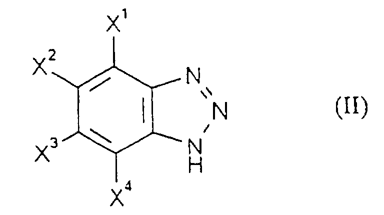 in welcher
X¹, X², X³ und X⁴ die oben angegebenen Bedeutungen haben,
mit Halogeniden der Formel
Hal-Y-R (III)
in welcher
R und Y die oben angegebenen Bedeutungen haben und
Hal für Chlor oder Brom steht,
gegebenenfalls in Gegenwart eines Säurebindemittels und gegebenenfalls in Gegenwart eines Verdünnungsmittels umsetzt und gegebenenfalls an die so erhaltenen Verbindungen der Formel (I) eine Säure oder ein Metallsalz addiert.

3. Mikrobizide Mittel, **gekennzeichnet durch** einen Gehalt an mindestens einem Benzotriazol der Formel (I) gemäß Anspruch 1 bzw. an einem Säureadditionssalz oder Metallsalz-Komplex eines Benzotriazols der Formel (I) neben Streckmitteln und/oder oberflachenaktiven Stoffen,

4. Verwendung von Benzotriazolen der Formel (I) gemäß Anspruch 1 bzw. von deren Säureadditionssalzen oder Metallsalz-Komplexen als Mikrobizide im Pflanzenschutz und im Materialschutz.

5. Verfahren zur Bekämpfung von unerwünschten Mikroorganismen im Pflanzenschutz und im Materialschutz, **dadurch gekennzeichnet, daß** man Benzotriazole der Formel (1) gemäß Anspruch 1 bzw. deren Säureadditionssalze oder Metallsalz-Komplexe auf die Mikroorganismen und/oder deren Lebensraums ausbringt.

6. Verfahren zur Herstellung von mikrobiziden Mitteln, **dadurch gekennzeichnet, daß** man Benzotriazole der Formel (I) gemäß Anspruch 1 bzw. deren Säureadditionssalze oder Metallsalz-Komplexe mit Streckmitteln und/oder oberflachenaktiven Stoffen vermischt.

## Claims

1. Benzotriazoles of the formula 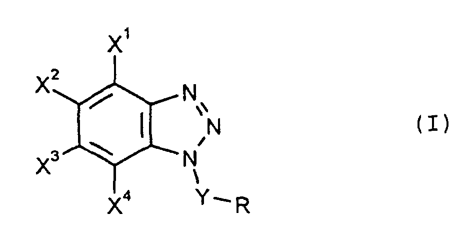 in which
X¹, X², X³ and X⁴, independently of each other, represent hydrogen, fluorine, chlorine, bromine, iodine, cyano, nitro, straight-chain or branched alkyl having 1 to 8 carbon atoms, straight-chain or branched halogenoalkyl having 1 to 6 carbon atoms and 1 to 13 identical or different halogen atoms, straight-chain or branched alkoxy having 1 to 8 carbon atoms, straight-chain or branched halogenoalkoxy having 1 to 6 carbon atoms and 1 to 13 identical or different halogen atoms, straight-chain or branched alkylthio having 1 to 8 carbon atoms, straight-chain or branched halogenoalkylthio having 1 to 6 carbon atoms and 1 to 13 identical or different halogen atoms, straight-chain or branched alkylsulphinyl having 1 to 8 carbon atoms, straight-chain or branched halogenoalkylsulphinyl having 1 to 6 carbon atoms and 1 to 13 identical or different halogen atoms, straight-chain or branched alkylsulphonyl having 1 to 8 carbon atoms, straight-chain or branched halogenoalkylsulphonyl having 1 to 6 carbon atoms and 1 to 13 identical or different halogen atoms, cycloalkyl having 3 to 6 carbon atoms which may optionally be monosubstituted to pentasubstituted, identically or differently, by halogen and/or alkyl having 1 to 4 carbon atoms, hydroxycarbonyl, alkylcarbonyl having 1 to 6 carbon atoms in the straight-chain or branched alkyl moiety, alkoxycarbonyl having 1 to 6 carbon atoms in the straight-chain or branched alkoxy moiety, cycloalkylcarbonyl having 3 to 6 carbon atoms in the cycloalkyl moiety, cycloalkoxycarbonyl having 3 to 6 carbon atoms in the cycloalkyl moiety, 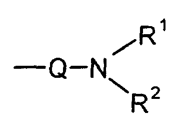 or -Z-R³,
in which
R¹ and R², independently of each other, represent hydrogen, straight-chain or branched alkyl having 1 to 6 carbon atoms, straight-chain or branched halogenoalkyl having 1 to 6 carbon atoms and 1 to 13 identical or different halogen atoms, straight-chain or branched alkoxyalkyl having 1 to 4 carbon atoms in the alkoxy moiety and 1 to 4 carbon atoms in the alkyl moiety, alkylcarbonyl having 1 to 6 carbon atoms in the straight-chain or branched alkyl moiety, aryl having 6 to 10 carbon atoms, arylcarbonyl having 6 to 10 carbon atoms in the aryl moiety, arylsulphonyl having 6 to 10 carbon atoms, arylaminocarbonyl having 6 to 10 carbon atoms in the aryl moiety or arylmethylsulphonyl having 6 to 10 carbon atoms in the aryl moiety, where each of the aryl radicals mentioned above can be monosubstituted to trisubstituted, identically of differently, by halogen, cyano, nitro, alkyl having 1 to 4 carbon atoms, alkoxy having 1 to 4 carbon atoms, alkylthio having 1 to 4 carbon atoms, halogenoalkyl having 1 to 4 carbon atoms and 1 to 5 identical or different halogen atoms, halogenoalkoxy having 1 to 4 carbon atoms and 1 to 5 identical or different halogen atoms, halogenoalkylthio having 1 to 4 carbon atoms and 1 to 5 identical or different halogen atoms, alkylsulphinyl having 1 to 4 carbon atoms, alkylsulphonyl having 1 to 4 carbon atoms, halogenoalkylsulphinyl having 1 to 4 carbon atoms and 1 to 5 identical or different halogen atoms and/or halogenoalkylsulphonyl having 1 to 4 carbon atoms and 1 to 5 identical or different halogen atoms,
or
R¹ and R² together with the nitrogen atom to which they are bound preferably represent a heterocyclic ring having 5 or 6 ring members which may optionally be monosubstituted to trisubstituted by alkyl having 1 to 4 carbon atoms and can additionally contain an oxygen atom or a C₁-C₄-alkylimino group,
Q represents a direct bond or a carbonyl group and
R³ represents aryl having 6 to 10 carbon atoms, where each of these radicals can be monosubstituted to trisubstituted, identically or differently, by halogen, cyano, nitro, alkyl having 1 to 4 carbon atoms, alkoxy having 1 to 4 carbon atoms, alkylthio having 1 to 4 carbon atoms, halogenoalkyl having 1 to 4 carbon atoms and 1 to 5 identical or different halogen atoms, halogenoalkoxy having 1 to 4 carbon atoms and 1 to 5 identical or. different halogen atoms, halogenoalkylthio having 1 to 4 carbon atoms and 1 to 5 identical or different halogen atoms, alkylsulphinyl having 1 to 4 carbon atoms, alkylsulphonyl having 1 to 4 carbon atoms, halogenoalkylsulphinyl having 1 to 4 carbon atoms and 1 to 5 identical or different halogen atoms and/or halogenoalkylsulphonyl having 1 to 4 carbon atoms and 1 to 5 identical or different halogen atoms, or
R³ represents a saturated or unsaturated heterocyclyl radical having 5 or 6 ring members and 1 to 3 hetero atoms, where these radicals can be monosubstituted to trisubstituted, identically or differently, by halogen, alkyl having 1 to 4 carbon atoms, alkoxy having 1 to 4 carbon atoms, halogenoalkyl having 1 to 4 carbon atoms and 1 to 5 identical or different halogen atoms, halogenoalkoxy having 1 to 4 carbon atoms and 1 to 5 identical or different halogen atoms, alkoxycarbonyl having 1 to 3 carbon atoms in the alkoxy moiety, cycloalkyl having 3 to 6 carbon atoms, cyano and/or nitro,
Z represents a direct bond or CH₂, O, S, SO, SO₂, CO, or
-CO-O-, where the oxygen atom is connected to the aryl or heterocyclyl radical, or
-SO₂-O-, where the sulphur atom is connected to the aryl or heterocyclyl radical, or
-S-CH₂-SO₂-, where the sulphur atom of the thio group is connected to the aryl or heterocyclyl radical,
or
X² and X³ together represent an alkylene chain having 3 or 4 members which is optionally monosubstituted to hexasubstituted by halogen, alkyl having 1 to 4 carbon atoms and/or halogenoalkyl having 1 to 4 carbon atoms and 1 to 9 halogen atoms, and in which alkylene chain one or two (non-adjacent) carbon atoms can be replaced by oxygen atoms, or
X² and X³ together represent a radical of the formula 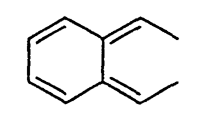
R represents a saturated or unsaturated heterocyclyl radical having 5 or 6 ring elements and 1 to 3 hetero atoms, where these radicals can be monosubstituted to trisubstituted, identically or differently, by halogen, cyano, nitro, hydroxyl, amino, formyl, carboxyl, carbamoyl, thiocarbamoyl, alkyl having 1 to 4 carbon atoms, alkoxy having 1 to 4 carbon atoms, halogenoalkyl having 1 to 4 carbon atoms, halogenoalkyl having 1 to 4 carbon atoms and 1 to 5 identical or different halogen atoms, halogenoalkoxy having 1 to 4 carbon atoms and 1 to 5 identical or different halogen atoms, alkoxycarbonyl having 1 to 4 carbon atoms in the alkoxy moiety, cycloalkyl having 3 to 6 carbon atoms, halogenoalkylsulphinyl having 1 to 4 carbon atoms and 1 to 5 identical or different halogen atoms, halogenoalkylsulphonyl having 1 to 4 carbon atoms and 1 to 5 identical or different halogen atoms, alkylamino having 1 to 4 carbon atoms, hydroxyalkylamino having 1 to 4 carbon atoms, dialkylamino having 1 to 4 carbon atoms in each alkyl group, alkylcarbonyl having 1 to 4 carbon atoms in the alkyl moiety, alkylcarbonylamino having 1 to 4 carbon atoms in the alkyl moiety, hydroxyiminoalkyl having 1 to 4 carbon atoms in the alkyl moiety, alkoxyiminoalkyl having 1 to 4 carbon atoms in the alkoxy moiety and 1 to 4 carbon atoms in the alkyl moiety, alkylcarbonyloxy having 1 to 4 carbon atoms in the alkyl moiety and/or halogenoalkylcarbonyloxy having 1 to 4 carbon atoms in the halogenoalkyl group and 1 to 5 identical or different halogen atoms, and where the heterocyclyl radicals can also contain oxo groups, and
Y represents -SO₂-,
and their acid addition salts and metal salt complexes.

2. Process for the preparation of benzotriazoles of the formula (I) according to Claim 1 and of their acid addition salts and metal salt complexes, **characterized in that** benzotriazoles of the formula 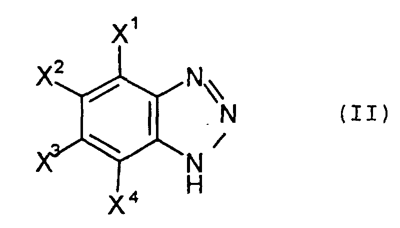 in which
X¹, X², X³ and X⁴ have the meanings given above,
are reacted with halides of the formula
Hal-Y-R (III)
in which
R and Y have the meanings given above and
Hal represents chlorine or bromine,
in the presence or absence of an acid acceptor and in the presence or absence of a diluent and, if appropriate, an acid or a metal salt is added to the compounds of the formula (I) thus obtained.

3. Microbicidal compositions, **characterized by** a content of at least one benzotriazole of the formula (I) according to Claim 1 or of an acid addition salt or metal salt complex of a benzotriazole of the formula (I) in addition to extenders and/or surface-active substances.

4. Use of benzotriazoles of the formula (I) according to Claim 1, or of their acid addition salts or metal salt complexes, as microbicides in crop protection and in material protection.

5. Process for controlling undesirable microorganisms in crop protection and in material protection, **characterized in that** benzotriazoles of the formula (I) according to Claim 1, or their acid addition salts or metal salt complexes, are applied to the microorganisms and/or their habitat.

6. Process for the preparation of microbicidal compositions, **characterized in that** benzotriazoles of the formula (I) according to Claim 1, or their acid addition salts or metal salt complexes, are mixed with extenders and/or surface-active substances.

## Revendications

1. Benzotriazoles de formule : 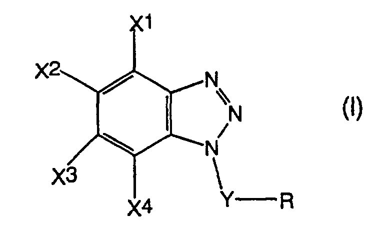 dans laquelle :
X¹, X², X³ et X⁴ représentent indépendamment l'un de l'autre, les atomes d'hydrogène, de fluor, de chlore, de brome, d'iode, les radicaux cyano, nitro, alcoyle linéaire ou ramifié, ayant 1 à 8 atomes de carbone, halogénoalcoyle linéaire ou ramifié, ayant 1 à 6 atomes de carbone et 1 à 13 atomes d'halogène identiques ou différents, alcoxy linéaire ou ramifié, ayant 1 à 8 atomes de carbone, ou halogénoalcoxy linéaire ou ramifié, ayant 1 à 6 atomes de carbone et 1 à 13 atomes d'halogène identiques ou différents, alcoylthio linéaire ou ramifié, ayant 1 à 8 atomes de carbone, halogénoalcoylthio linéaire ou ramifié, ayant 1 à 6 atomes de carbone et 1 à 13 atomes d'halogène identiques ou différents, alcoylsulfinyle linéaire ou ramifié, ayant 1 à 8 atomes de carbone, halogénoalcoylsulfinyle linéaire ou ramifié, ayant 1 à 6 atomes de carbone et 1 à 13 atomes d'halogène identiques ou différents, alcoylsulfonyle linéaire ou ramifié, ayant 1 à 8 atomes de carbone, halogénoalcoylsulfonyle linéaire ou ramifié, ayant 1 à 6 atomes de carbone et 1 à 13 atomes d'halogène identiques ou différents, cycloalcoyle ayant 3 à 6 atomes de carbone, éventuellement substitué une à cinq fois, de manière identique ou différente, par un atome d'halogène ou le radical alcoyle ayant 1 à 4 atomes de carbone, les radicaux hydroxycarbonyle, alcoylcarbonyle ayant 1 à 6 atomes de carbone dans la partie alcoyle linéaire ou ramifiée, alcoxycarbonyle ayant 1 à 6 atomes de carbone dans la partie alcoxy linéaire ou ramifiée, cycloalcoylcarbonyle ayant 3 à 6 atomes de carbone dans la partie cycloalcoyle, cycloalcoxycarbonyle ayant 3 à 6 atomes de carbone dans la partie cycloalcoxy, les radicaux -Q-NR¹R² ou Z-R³,
où
R¹ et R² représentent indépendamment l'un de l'autre, l'atome d'hydrogène, les radicaux alcoyle linéaire ou ramifié, ayant 1 à 6 atomes de carbone, halogénoalcoyle linéaire ou ramifié, ayant 1 à 6 atomes de carbone et 1 à 13 atomes d'halogène identiques ou différents, alcoxyalcoyle linéaire ou ramifié, ayant 1 à 4 atomes de carbone dans la partie alcoxy et 1 à 4 atomes de carbone dans la partie alcoyle, alcoylcarbonyle ayant 1 à 6 atomes de carbone dans la partie alcoyle linéaire ou ramifiée, aryle ayant 6 à 10 atomes de carbone, arylcarbonyle ayant 6 à 10 atomes de carbone dans la partie aryle, arylsulfonyle ayant 6 à 10 atomes de carbone, arylaminocarbonyle ayant 6 à 10 atomes de carbone dans la partie aryle, ou arylméthylsulfonyle ayant 6 à 10 atomes de carbone dans la partie aryle, où chacun des restes aryle cités ci-dessus peut être substitué une à trois fois, de manière identique ou différente, par un atome d'halogène, les radicaux cyano, nitro, alcoyle ayant 1 à 4 atomes de carbone, alcoxy ayant 1 à 4 atomes de carbone, alcoylthio ayant 1 à 4 atomes de carbone, halogénoalcoyle ayant 1 à 4 atomes de carbone et 1 à 5 atomes d'halogène identiques ou différents, halogénoalcoxy ayant 1 à 4 atomes de carbone et 1 à 5 atomes d'halogène identiques ou différents, halogénoalcoylthio ayant 1 à 4 atomes de carbone et 1 à 5 atomes d'halogène identiques ou différents, alcoylsulfinyle ayant 1 à 4 atomes de carbone, alcoylsulfonyle ayant 1 à 4 atomes de carbone, halogénoalcoylsulfinyle ayant 1 à 4 atomes de carbone et 1 à 5 atomes d'halogène identiques ou différents et/ou halogénoalcoylsulfonyle ayant 1 à 4 atomes de carbone et 1 à 5 atomes d'halogène identiques ou différents, ou
R¹ et R² représentent également avec l'atome d'azote auquel ils sont liés, de préférence un hétérocycle ayant 5 ou 6 membres dans le cycle, éventuellement substitué une à trois fois par un radical alcoyle ayant 1 à 4 atomes de carbone, lequel peut encore contenir un atome d'oxygène ou un radical (alcoyl en C₁-C₄)imino,
Q représente une liaison directe ou le radical carbonyle, et
R³ représente un radical aryle ayant 6 à 10 atomes de carbone, où chacun de ces restes peut être substitué une à trois fois, de manière identique ou différente, par un atome d'halogène, les radicaux cyano, nitro, alcoyle ayant 1 à 4 atomes de carbone, alcoxy ayant 1 à 4 atomes de carbone, alcoylthio ayant 1 à 4 atomes de carbone, halogénoalcoyle ayant 1 à 4 atomes de carbone et 1 à 5 atomes d'halogène identiques ou différents, halogénoalcoxy ayant 1 à 4 atomes de carbone et 1 à 5 atomes d'halogène identiques ou différents, halogénoalcoylthio ayant 1 à 4 atomes de carbone et 1 à 5 atomes d'halogène identiques ou différents, alcoylsulfinyle ayant 1 à 4 atomes de carbone, alcoylsulfonyle ayant 1 à 4 atomes de carbone, halogénoalcoylsulfinyle ayant 1 à 4 atomes de carbone et 1 à 5 atomes d'halogène identiques ou différents et/ou halogénoalcoylsulfonyle ayant 1 à 4 atomes de carbone et 1 à 5 atomes d'halogène identiques ou différents, ou
R³ représente un reste hétérocyclyle saturé ou insaturé, ayant 5 ou 6 membres dans le cycle et 1 à 3 hétéroatomes, où ces restes peuvent être substitués une à trois fois, de manière identique ou différente par un atome d'halogène, les radicaux alcoyle ayant 1 à 4 atomes de carbone, alcoxy ayant 1 à 4 atomes de carbone, halogénoalcoyle ayant 1 à 4 atomes de carbone et 1 à 5 atomes d'halogène identiques ou différents, halogénoalcoxy ayant 1 à 4 atomes de carbone et 1 à 5 atomes d'halogène identiques ou différents, alcoxycarbonyle ayant 1 à 3 atomes de carbone dans la partie alcoxy, cycloalcoyle ayant 3 à 6 atomes de carbone, cyano et/ou nitro,
Z représente une liaison directe ainsi que les radicaux CH₂, O, S, SO, SO₂, CO ou
le radical -CO-O-, où l'atome d'oxygène est relié à un reste aryle ou hétérocyclyle, ou
le radical -SO₂-O-, où l'atome de soufre est relié à un reste aryle ou hétérocyclyle, ou
le radical -S-CH₂-SO₂-, où l'atome de soufre du radical thio est relié à un reste aryle ou hétérocyclyle, ou
X² et X³ représentent ensemble une chaine alcoylène ayant 3 ou 4 chainons, éventuellement une à six fois substituée par un atome d'halogène, les radicaux alcoyle ayant 1 à 4 atomes de carbone et/ou halogénoalcoyle ayant 1 à 4 atomes de carbone et 1 à 9 atomes d'halogène, dans laquelle un ou deux atomes de carbone (non adjacents) peuvent être remplacés par l'atome d'oxygène, ou
X² et X³ représentent ensemble un reste de formule : 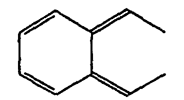
R représente un reste hétérocyclyle saturé ou insaturé, ayant 5 ou 6 membres dans le cycle et 1 à 3 hétéroatomes, où ce reste peut être substitué une à trois fois, de manière identique ou différente, par un atome d'halogène, les radicaux cyano, nitro, hydroxy, amino, formyle, carboxy, carbamoyle, thiocarbamoyle, alcoyle ayant 1 à 4 atomes de carbone, alcoxy ayant 1 à 4 atomes de carbone, halogénoalcoyle ayant 1 à 4 atomes de carbone et 1 à 5 atomes d'halogène identiques ou différents, halogénoalcoxy ayant 1 à 4 atomes de carbone et 1 à 5 atomes d'halogène identiques ou différents, alcoxycarbonyle ayant 1 à 4 atomes de carbone dans la partie alcoxy, cycloalcoyle ayant 3 à 6 atomes de carbone, halogénoalcoylsulfinyle ayant 1 à 4 atomes de carbone et 1 à 5 atomes d'halogène identiques ou différents, halogénoalcoylsulfonyle ayant 1 à 4 atomes de carbone et 1 à 5 atomes d'halogène identiques ou différents, alcoylamino ayant 1 à 4 atomes de carbone, hydroxyalcoylamino ayant 1 à 4 atomes de carbone, dialcoylamino ayant 1 à 4 atomes de carbone dans chaque radical alcoyle, alcoylcarbonyle ayant 1 à 4 atomes de carbone dans la partie alcoyle, alcoylcarbonylamino ayant 1 à 4 atomes de carbone dans la partie alcoyle, hydroximinoalcoyle ayant 1 à 4 atomes de carbone dans la partie alcoyle, alcoximinoalcoyle ayant 1 à 4 atomes de carbone dans la partie alcoxy et 1 à 4 atomes de carbone dans la partie alcoyle, alcoylcarbonyloxy ayant 1 à 4 atomes de carbone dans la partie alcoyle et/ou halogénoalcoylcarbonyloxy ayant 1 à 4 atomes de carbone dans la partie halogénoalcoyle et 1 à 5 atomes d'halogène identiques ou différents, et où les restes hétérocyclyle peuvent également contenir des radicaux oxo, et
Y représente le radical -SO₂-,
ainsi que leurs sels d'addition d'acide et complexes de sel métallique.

2. Procédé de préparation de benzotriazoles de formule (I) suivant la revendication 1, ainsi que de leurs sels d'addition d'acide et de leurs complexes de sel métallique, **caractérisé en ce que** l'on fait réagir les benzotriazoles de formule : 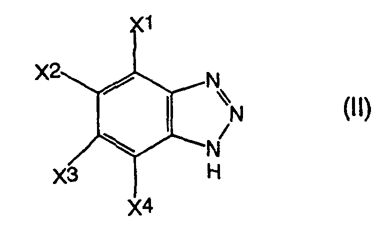 dans laquelle X¹, X², X³ et X⁴ ont les significations données ci-dessus,
avec des halogénures de formule :
Hal - Y - R (III)
dans laquelle R et Y ont les significations données ci-dessus, et
Hal représente l'atome de chlore ou de brome,
le cas échéant en présence d'un agent liant les acides et le cas échéant, en présence d'un agent de dilution, et le cas échéant, on additionne un acide ou un sel métallique au composé de formule (I) ainsi obtenu.

3. Agent microbiocide, **caractérisé par** une teneur en au moins un benzotriazole de formule (I) suivant la revendication 1 ou en un sel d'addition d'acide ou complexe de sel métallique d'un benzotriazole de formule (I), ainsi qu'un agent diluant et/ou un agent tensioactif.

4. Utilisation des benzotriazoles de formule (I) suivant la revendication 1, ou de leurs sels d'addition d'acide et de leurs complexes de sel métallique, comme microbiocide dans la protection des végétaux et dans la protection des matériaux.

5. Procédé pour lutter contre des microorganismes non souhaités dans la protection des végétaux et dans la protection des matériaux, **caractérisé en ce que** l'on applique des benzotriazoles de formule (I) suivant la revendication 1, ou leurs sels d'addition d'acide ou leurs complexes de sel métallique, sur les microorganismes et/ou leur lieu de développement.

6. Procédé de préparation d'agents microbiocides, **caractérisé en ce que** l'on mélange les benzotriazoles de formule (I) suivant la revendication 1 ou leurs sels d'addition d'acide ou complexes de sel métallique avec un agent diluant et/ou un agent tensioactif.
